# EUROPEAN PATENT SPECIFICATION

(11) **EP 4 436 398 B1**
(45) Date of publication and mention of the grant of the patent: **12.08.2026**
(21) Application number: 22830297.2
(22) Date of filing: 22.11.2022
(51) Int. Cl.: A23D 9/00, A23L 33/12, A23L 33/00, A61K 31/202, C12N 1/12, C12P 7/6427

(54) **COMPOSITIONS HAVING HIGH LEVELS OF DPA (N-3) AND METHODS FOR PRODUCING**
ZUBEREITUGEN ENTHALTEND HOHE MENGEN AN DPA (N-3) UND HERSTELLUNGSVERFAHREN
COMPOSITIONS COMPRENANT DE HAUTS NIVEAUX DE DPA (N-3) ET PROCEDEE DE PREPARATION

(30) Priority: 24.11.2021 US 202163282712 P; 24.11.2021 US 202163282714 P; 08.03.2022 US 202263317797 P; 08.03.2022 US 202263317805 P
(43) Date of publication of application: 02.10.2024
(60) Divisional application: 26189569.2
(73) Proprietor: DSM IP Assets B.V., 6221 BE Maastricht (NL)
(72) Inventor: ZIRKLE, Ross E., Columbia, Maryland 21045 (US); MAY, Oliver, Columbia, Maryland 21045 (US)
(74) Representative: Hoyng Rokh Monegier B.V.
(86) International application number: PCT/IB2022/061295
(87) International publication number: WO 2023/094996

(56) References cited:
- CN-A- 103 642 860
- US-A1- 2015 203 826
- US-A1- 2016 177 255
- US-A1- 2017 216 239
- US-A1- 2021 047 663
- DULCE MARTINS ET AL: "Alternative Sources of n-3 Long-Chain Polyunsaturated Fatty Acids in Marine Microalgae", MARINE DRUGS, vol. 11, no. 7, 27 June 2013 (2013-06-27), pages 2259 - 2281, XP055185025, DOI: 10.3390/md11072259
- HOJSAK IVA ET AL: "Young Child Formula: A Position Paper by the ESPGHAN Committee on Nutrition", JOURNAL OF PEDIATRIC GASTROENTEROLOGY AND NUTRITION, vol. 66, no. 1, 1 January 2018 (2018-01-01), US, pages 177 - 185, XP093018007, ISSN: 0277-2116, DOI: 10.1097/MPG.0000000000001821

## Description

### BACKGROUND

It is desirable to increase the dietary intake of beneficial long chain polyunsaturated fatty acids (LC-PUFAs), including for example, omega-3 long chain polyunsaturated fatty acids (omega-3 LC-PUFAs), and omega-6 long chain polyunsaturated fatty acids (omega-6 LC-PUFAs) for human infants and toddlers, for adult humans, and for other animals. As used herein, reference to a long chain polyunsaturated fatty acid or LC-PUFA, refers to a polyunsaturated fatty acid having 18 or more carbons. Recognition of clinical benefits attributed to omega-6 and omega-3 long-chain polyunsaturated fatty acids (LC-PUFAs) has stimulated efforts to increase the use of these fatty acids in infant diets (Carlson and Forsythe 2001 Curr. Op. Clin. Nutr. Metab. Care 4, 123-126; Birch et al. 2000 Develop. Med. Child Neuro. 42, 174-181). Furthermore, the clinical benefits of omega-3 LC-PUFA for maternal supplements, and other types of nutritional supplements and foods are also well recognized (Barclay and Van Elswyk 2000, FUNCTIONAL FOODS 2000, Angus, F. and Miller C, eds., pp. 60-67, Leatherhead Publishing, Surrey) The addition of the (n-3) fatty acids DHA and EPA to e.g., infant formulas, using oils from labyrinthulomycete microorganisms, which also comprise (n-6)DPA, is disclosed in US 2016/177255 A1.

Fatty acids are classified based on the length and saturation characteristics of the carbon chain. As used herein, fatty acids include fatty acids in various forms, including but not limited to triacylglycerols, diacylglycerols, monoacylglycerols, phospholipids, free fatty acids, esterified fatty acids, and natural or synthetic derivative forms of these fatty acids (e.g., calcium salts of fatty acids, ethyl esters, etc). Short chain fatty acids have 2 to about 7 carbons and are typically saturated. Medium chain fatty acids have from about 8 to about 17 carbons and may be saturated or unsaturated. Long chain fatty acids have from 18 to 24 or more carbons and may also be saturated or unsaturated, and there may be one or more points of unsaturation, giving rise to the terms "monounsaturated" and "polyunsaturated," respectively. Long chain PUFAs (LC-PUFAs) are of particular interest in the present invention.

LC-PUFAs are categorized according to the number and position of double bonds in the fatty acids according to a well understood nomenclature. There are two common series or families of LC-PUFAs, depending on the position of the double bond closest to the methyl end of the fatty acid: the n-3 (or ω-3 or omega-3) series contains a double bond at the third carbon, while the n-6 (or ω-6 or omega-6) series has no double bond until the sixth carbon. Thus, docosahexaenoic acid ("DHA") has a chain length of 22 carbons with 6 double bonds beginning with the third carbon from the methyl end and is designated "22:6 n-3". Other important omega-3 LC-PUFAs include eicosapentaenoic acid ("EPA") which is designated "20:5 n-3" and docosapentaenoic acid n-3 ("DPA(n-3)") which is designated "22:5 n-3." In addition, omega-6 LC-PUFAs may be used in connection with the present invention. For example, arachidonic acid ("ARA") which is designated "20:4 n-6" and docosapentaenoic acid n-6 ("DPAn-6") which is designated "22:5 n-6" are suitable.

De novo or "new" synthesis of omega-3 fatty acids and omega-6 fatty acids does not occur in the human body. The precursor fatty acid for the omega-3 and omega-6 fatty acids are alpha-linolenic acid (18:3n-3) and linoleic acid (18:2n-6), respectively. These fatty acids are essential fatty acids and must be consumed in the diet because humans cannot synthesize them. Humans cannot insert double bonds closer to the omega end than the seventh carbon atom counting from that end of the molecule. However, the body can convert alpha-linolenic acid and linoleic acid to LC PUFAs such as DHA and ARA, respectively, although at very low efficiency. All metabolic conversions occur without altering the omega end of the molecule that contains the omega-3 and omega-6 double bonds. Consequently, omega-3 and omega-6 acids are two separate families of fatty acids since they are not interconvertible in the human body.

Both term and preterm infants can synthesize the LC-PUFAs from the respective essential fatty acids, but controversy has centered around the fact that breastfed infants have higher plasma concentrations of these LC-PUFAs than formula-fed infants. This information could be interpreted to imply that formula-fed infants cannot synthesize enough of these fatty acids to meet ongoing needs, though the plasma content of DHA and ARA is only a very small fraction of the total fatty acid pool available in other tissues. It has been demonstrated that the addition of DHA to infant formula improves infant visual function, and that the addition of both DHA and ARA improves cognitive development and facilitates normal infant growth. Sources of oils containing both DHA and DPA(n-6), and oil containing ARA, have been developed for nutritional use and these have been suggested for use in infant formula to better match the LC-PUFA profile found in human breast milk.

Arachidonic acid, along with its elongation products docosatetraenoic acid and docosapentaenoic acid, has been suggested for inclusion in infant diets along with docosahexaenoic acid in recognition of their natural occurrence in human breast milk (Specter 1994).

A number of organizations provide recommendations for levels of LC-PUFAs in infant formulas. For example, the International Society for the Study of Fatty Acids and Lipids (ISSFAL) recommended in 1994 that infant formulas provide 60-100 mg/kg/day as preformed arachidonic acid and its associated long chain omega-6 forms (22:4(n-6) and 22:5(n-6)) (ISSFAL Board of Directors, ISSFAL Newsletter :4-5 (1994)). ISSFAL made the following recommendations for LC-PUFAs in infant formula in 1999 in order to ensure adequate intake of the LC-PUFAs: linoleic acid, 18:2n-6, 10%; α-linolenic acid, 18:3, 1.50%; arachidonic acid, 20:4n-6, 0.50%; docosahexaenoic acid, 22:6n-3, 0.35%; eicosapentaenoic acid, 20:5n-3, 0.10%.

Established Recommended Daily Intakes (RDIs) for the long chain omega-3 fatty acids (DHA/DPA/EPA) range from 200 mg/day (COMA (Committee on Medical Aspects of Food Policy) (1994). Annual Report. London: Department of Health) to 1.2 g/day (Nordic Council of Ministers (1989). Nordic Nutrition Recommendations, Second Edition). These RDIs represent a range of DHA/EPA intakes from 3 to 20 mg DHA+EPA/kg/day for adults. Studies from Europe indicate the average daily intake of DPA(n-3) in adults can range from 25 mg/day to 75 mg/day (Leng et al. 2017 Int. J. COPD. 12, 3171-3181). In contrast, Greenlandic Inuit are estimated to consume between 1.7 and 4.0 g DPA/day due to their high intake of marine-based foods and lipids (Bang et al. 1980 Am. J. Clin. Nutr. 33, 2657-2661).

For pregnant and lactating women, the U.S. Food and Drug Administration recommends consumption of 8-12 ounces of seafood, such as salmon, per week. Given that Atlantic farmed salmon contains roughly 333 mg/100 g DPA, consumption of 12 ounces of salmon per week would provide an average of 162 mg/day DPA (Cladis et al. 2014 Lipids 49, 1005-1018). However, actual maternal DPA intake in pregnant and lactating women in Western countries is fairly low with various studies reporting between about 18 mg/day and 35 mg/day (Denomme et al. 2005 J. Nutr. 135, 206-211; Garneau et al. 2012 Nutr. J. 11, 1-6; Field et al. 2014 11th ISSFAL Congress Book of Abstract, p. 24).

For infants, a reference intake for comparison purposes can be gleaned from the data on the DPA(n-3) content in human breast milk. DPA(n-3) content as a percentage of the total fat in human breast milk has been reported to range from 0.06 to 0.75% in Western countries, with higher levels generally seen in colostrum (0.11-0.75%) versus mature breast milk (0.06 to 0.52%). In one study, breast milk fat was shown to contain 0.22% of DHA, 0.17% DPA, and 0.04% EPA. Assuming infant daily consumption of breast milk is 750 mL/day, body weight of 3.5 kg, and average fat content of breast milk of 4%, DPA daily intake of breast-fed infants ranges from about 18 to about 156 mg/day (about 5.1-44.6 mg/kg/day). This would equate to about 357-3122 mg DPA/day for a 70 kg adult (Li et al. 2016 Eur. J. Lipid Sci. Technol. 118, 1692-1701).

For formula-fed infants, consumption of DPA is difficult to ascertain. Since there is no recommendation for DPA content in formulas, infant formulas are not typically supplemented with DPA. It has been reported that formula-fed infants without LC-PUFA (n-3) supplementation have significantly lower plasma DPA and DHA than breast-fed infants (Socha et al. 1998 Acta Paediatr. 87, 278-283; Woltil et al. 1995 Am. J. Clin. Nutr. 62, 943-949).

Although the role of DHA in prenatal and postnatal development is well-established and infant formulas are regularly supplemented with DHA, it is important to note that levels of DPA(n-3) are nearly the same as DHA in human breast milk. DPA(n-3) is the second most abundant n-3 LC-PUFA in the brain (behind DHA). The specific role for DPA(n-3) is still under investigation, but it could play a role in neural development and function. It is noted that the high level of DPA(n-3) intake in infants (in terms of mg/kg body weight) occurs during the period when brain and neural tissues are in rapid development.

DPA(n-3) is known to be able to directly convert to DHA and retro-convert to eicosapentaenoic acid (EPA), suggesting also that it may serve as a reservoir for these n-3 LC-PUFAs in the body.

ARA is generally the LC-PUFA added in the highest concentration to infant formula. Current recommendations from health and regulatory organizations suggest that ARA and DHA should be added to infant formula in an approximate ratio of about 2:1-1:1 (ARA:DHA). It is known that high dietary intake of omega-3 LC-PUFAs such as DHA results in an increase in DHA content, but also reduces plasma levels of ARA. Thus, ARA is added to infant formulas at these levels to compensate for the decline in ARA plasma levels resulting from DHA administration. Infant formulas containing LC-PUFAs can be more expensive than standard infant formulas, due to the added cost of the LC-PUFA ingredients.

It would be desirable to enrich human and animal compositions, including infant formula, in a manner that provides the benefits of supplementation of DPA(n-3) and is economically feasible. These and other needs are answered by the present invention.

The solution to this technical problem is provided by the embodiments characterized in the claims.

### BRIEF SUMMARY

The invention provides an infant formula composition, wherein the composition comprises long chain polyunsaturated fatty acids (LC-PUFAs) and wherein, when ready for consumption by the infant, the LC-PUFAs comprise docosapentaenoic acid n-3 (DPA(n-3)) and docosahexaenoic acid (DHA) in a ratio of DPA(n-3):DHA greater than 0.2, and wherein the LC-PUFAs are supplied in a source oil, wherein the source oil comprises DPA(n-3) in an amount of at least about 7% by weight.

In certain embodiments, the ratio of DPA(n-3):DHA is greater than 0.2:1. In some embodiments, the ratio of DPA(n-3):DHA is greater than about 0.3:1, greater than about 0.4:1, greater than about 0.5:1, greater than about 0.6:1, greater than about 0.7:1, or greater than about 0.8:1.

In certain embodiments, the DPA(n-3):DHA ratio is from about 0.2:1 to about 1:1, from about 0.3:1 to about 1:1, from about 0.4:1 to about 1:1, from about 0.5:1 to about 1:1, from about 0.6:1 to about 1:1, from about 0.7:1 to about 1:1, from about 0.2 to about 0.9, from about 0.2 to about 0.8, from about 0.3 to about 0.8, about 0.2:1, about 0.3:1, about 0.4:1, about 0.5:1, about 0.6:1, about 0.7:1, about 0.8:1, about 0.9:1, or about 1:1.

In certain embodiments, the DHA:DPA(n-3):EPA ratio in the composition is 0.01 to 1 to 0.01-0.70, preferably from 0.20 to 1 to 0.20-0.70, or 0.20-0.70 to 1 to 0.20, for example: 0.2:1:0.2; 0.2:1:0.25; 0.2:1:0.3; 0.2:1:0.35; 0.2:1:0.4; 0.2:1:0.45; 0.2:1:0.5; 0.2:1:0.55; 0.2:1:0.6; 0.2:1:0.65; 0.2:1:0.7; 0.25:1:0.2; 0.25:1:0.25; 0.25:1:0.3; 0.25:1:0.35; 0.25:1:0.4; 0.25:1:0.45; 0.25:1:0.5; 0.25:1:0.55; 0.25:1:0.6; 0.25:1:0.65; 0.25:1:0.7; 0.3:1:0.2; 0.3:1:0.25; 0.3:1:0.3; 0.3:1:0.35; 0.3:1:0.4; 0.3:1:0.45; 0.3:1:0.5; 0.3:1:0.55; 0.3:1:0.6; 0.3:1:0.65; 0.3:1:0.7; 0.35:1:0.2; 0.35:1:0.25; 0.35:1:0.3; 0.35:1:0.35; 0.35:1:0.4; 0.35:1:0.45; 0.35:1:0.5; 0.35:1:0.55; 0.35:1:0.6; 0.35:1:0.65; 0.35:1:0.7; 0.4:1:0.2; 0.4:1:0.25; 0.4:1:0.3; 0.4:1:0.35; 0.4:1:0.4; 0.4:1:0.45; 0.4:1:0.5; 0.4:1:0.55; 0.4:1:0.6; 0.4:1:0.65; 0.4:1:0.7; 0.45:1:0.2; 0.45:1:0.25; 0.45:1:0.3; 0.45:1:0.35; 0.45:1:0.4; 0.45:1:0.45; 0.45:1:0.5; 0.45:1:0.55; 0.45:1:0.6; 0.45:1:0.65; 0.45:1:0.7; 0.5:1:0.2; 0.5:1:0.25; 0.5:1:0.3; 0.5:1:0.35; 0.5:1:0.4; 0.5:1:0.45; 0.5:1:0.5; 0.5:1:0.55; 0.5:1:0.6; 0.5:1:0.65; 0.5:1:0.7; 0.55:1:0.2; 0.55:1:0.25; 0.55:1:0.3; 0.55:1:0.35; 0.55:1:0.4; 0.55:1:0.45; 0.55:1:0.5; 0.55:1:0.55; 0.55:1:0.6; 0.55:1:0.65; 0.55:1:0.7; 0.6:1:0.2; 0.6:1:0.25; 0.6:1:0.3; 0.6:1:0.35; 0.6:1:0.4; 0.6:1:0.45; 0.6:1:0.5; 0.6:1:0.55; 0.6:1:0.6; 0.6:1:0.65; 0.6:1:0.7; 0.65:1:0.2; 0.65:1:0.25; 0.65:1:0.3; 0.65:1:0.35; 0.65:1:0.4; 0.65:1:0.45; 0.65:1:0.5; 0.65:1:0.55; 0.65:1:0.6; 0.65:1:0.65; 0.65:1:0.7; 0.7:1:0.2; 0.7:1:0.25; 0.7:1:0.3; 0.7:1:0.35; 0.7:1:0.4; 0.7:1:0.45; 0.7:1:0.5; 0.7:1:0.55; 0.7:1:0.6; 0.7:1:0.65; or 0.7:1:0.7. In certain embodiments, the DHA:DPA(n-3):EPA ratio in the composition or infant formula composition is 1:0.5:0.3.

The long chain polyunsaturated fatty acids in the infant formula composition are supplied in a source oil, wherein the source oil comprises DPA(n-3) in an amount of at least about 7% by weight. In certain embodiments, the source oil comprises long chain n-3 fatty acids comprising at least about 8% 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, or 35% by weight DPA(n-3).

In certain embodiments, the infant formula composition comprises eicosapentaenoic acid (EPA) in an amount less than about 3 mg/L. In certain embodiments, the infant formula composition comprises EPA in an amount less than about 60 mg/L. In other embodiments, the infant formula composition comprises from about 15 mg/L to about 30 mg/L EPA. In some embodiments, the EPA:DHA ratio is provided in a ratio of up to 1: 1.

In certain embodiments, the infant formula composition is formulated to provide at least about 5 mg/kg/day DPA(n-3) when administered to an infant. In some embodiments, the infant formula composition is formulated to provide from about 5 mg/kg/day DPA(n-3) to about 45 mg/kg/day DPA(n-3) when administered to an infant.

In some embodiments of all of the foregoing embodiments, the DPA(n-3) and/or DHA is from a source selected from the group consisting of a plant, an oilseed, a microorganism, an animal, and mixtures of the foregoing. In some embodiments, the microorganism is selected from the group consisting of algae, bacteria, fungi and protists. In some embodiments, the microorganism is a protist within the Ichthyosporea class. In some embodiments, the microorganism is *Sphaeroforma arctica.* In other embodiments, the microorganism selected from the group consisting of Thraustochytriales, dinoflagellates, and Mucorales. In some embodiments, the microorganism is selected from the group consisting of Schizochytrium, Thraustochytrium, Crypthecodinium, and Mortierella.

In other embodiments, the source is selected from the group consisting of genetically modified plant and genetically modified oilseed selected from the group consisting of soybean, corn, safflower, sunflower, canola, flax, peanut, mustard, rapeseed, chickpea, cotton, lentil, white clover, olive, palm, borage, evening primrose, linseed and tobacco and mixtures thereof.

In still other embodiments, the source is selected from the group consisting of a genetically modified plant, a genetically modified oilseed, and a genetically modified microorganism, wherein the genetic modification comprises the introduction of a polyketide synthase gene or a portion thereof. As will be appreciated by one skilled in the art, a genetically modified oilseed is part of a genetically modified plant.

In some embodiments, the ratio of DPA(n-3):DHA in the composition is obtained by mixing plant, fish or microbial oils with a second microbial oil having a DHA:DPA(n-3) ratio of <0.7:1. In some embodiments, the second microbial oil has a DHA:DPA(n-3):EPA ratio of <0.7: 1:<0.7. In some embodiments, the second microbial oil is produced by one or more microorganisms described herein. For example, the second microbial oil may be produced by a microorganism selected from the group consisting of algae, bacteria, fungi and protists. In some embodiments, the microorganism is a protist within the Ichthyosporea class such as, for example, Sphaeroforma arctica. In some embodiments, the microorganism is selected from the group consisting of Thraustochytriales, dinoflagellates, and Mortierella. In some embodiments, the microorganism is selected from the group consisting of Schizochytrium, Thraustochytrium, and Crypthecodinium.

In further embodiments, a microbial oil is provided comprising DPA(n-3) in an amount of at least about 7% by weight, wherein the DPA(n-3):DHA ratio is greater than 0.2. In further embodiments, a microbial oil is provided comprising DPA(n-3) in an amount of at least about 7% by weight, wherein the DPA(n-3):DHA ratio is greater than 1.

In some embodiments, the microbial oil comprises DPA(n-3) in an amount between about 7 to about 11% by weight or about 8 to about 10% by weight.

In certain embodiments, the DPA(n-3):DHA ratio is from about 0.2:1 to about 1:1, from about 0.3:1 to about 1:1, from about 0.4:1 to about 1:1, from about 0.5:1 to about 1:1, from about 0.6:1 to about 1:1, from about 0.7:1 to about 1:1, from about 0.2 to about 0.9, from about 0.2 to about 0.8, from about 0.3 to about 0.8, about 0.2:1, about 0.3:1, about 0.4:1, about 0.5:1, about 0.6:1, about 0.7:1, about 0.8:1, about 0.9:1, or about 1:1.

In some embodiments, the microbial oil comprises long chain n-3 fatty acids comprising at least about 7% DPA(n-3) by weight of the total fatty acids. In some embodiments, the microbial oil comprises long chain n-3 fatty acids comprising at least about 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, or 35% DPA(n-3) by weight. In some embodiments, the microbial oil comprises between about 7% to about 50% DPA(n-3) by weight, between about 7% to about 35% DPA(n-3) by weight, between about 7% to about 20% DPA(n-3) by weight, between about 7% to about 15% DPA(n-3) by weight, between about 7% to about 14% DPA(n-3) by weight, between about 7% to about 13% DPA(n-3) by weight, between about 7% to about 12% DPA(n-3) by weight, between about 7% to about 11% DPA(n-3) by weight, between about 7% to about 10% DPA(n-3) by weight, or between about 7% to about 9% DPA(n-3) by weight. In some embodiments, the microbial oil comprises between about 8% to about 15% DPA(n-3) by weight, between about 8% to about 14% DPA(n-3) by weight, between about 8% to about 13% DPA(n-3) by weight, between about 8% to about 12% DPA(n-3) by weight, between about 8% to about 11% DPA(n-3) by weight, or between about 8% to about 10% DPA(n-3) by weight. All weight percentages are based on by weight % of the total fatty acids.

In some embodiments, the microbial oil comprises less than about 5% DHA by weight, less than about 4.5% DHA by weight, less than about 4% DHA by weight, less than about 3.5% DHA by weight, less than about 3% DHA by weight, less than about 2.5% DHA by weight, or less than about 2% DHA by weight. In some embodiments, the microbial oil comprises between about 1% to about 5% DHA by weight, between about 1% to about 4% DHA by weight, or between about 2% to about 4% DHA by weight. All weight percentages are based on by weight % of the total fatty acids.

In some embodiments, the microbial oil comprises less than about 5% EPA by weight, less than about 4.5% EPA by weight, less than about 4% EPA by weight, less than about 3.5% EPA by weight, less than about 3% EPA by weight, less than about 2.5% EPA by weight, or less than about 2% EPA by weight. In some embodiments, the microbial oil comprises between about 1% to about 5% EPA by weight, between about 2% to about 5% EPA by weight, or between about 3% to about 5% EPA by weight. All weight percentages are based on by weight % of the total fatty acids.

In some embodiments, the microbial oil comprises a total fat content greater than about 20% by weight, greater than about 21% by weight, greater than about 22% by weight, greater than about 23% by weight, greater than about 24% by weight, greater than about 25% by weight, greater than about 26% by weight, greater than about 27% by weight, greater than about 28% by weight, greater than about 29% by weight, greater than about 30% by weight, greater than about 31% by weight, greater than about 32% by weight, greater than about 33% by weight, greater than about 34% by weight, or greater than about 35% by weight. In some embodiments, the microbial oil comprises a total fat content between about 20 to about 40% by weight, between about 20 to about 35% by weight, between about 25 to about 40% by weight, or between about 25 to about 35% by weight.

In some embodiments, the microbial oil is produced by a microorganism selected from the group consisting of algae, bacteria, fungi and protists. In some embodiments, the microorganism is a protist within the Ichthyosporea class. In some embodiments, the microorganism is *Sphaeroforma arctica.* In other embodiments, the microorganism selected from the group consisting of Thraustochytriales, dinoflagellates, and Mucorales. In some embodiments, the microorganism is selected from the group consisting of Schizochytrium, Thraustochytrium, Crypthecodinium, and Mortierella. In some embodiments, the microorganism is a genetically modified microorganism, wherein the genetic modification comprises the introduction of a polyketide synthase gene or a portion thereof.

Compositions comprising oils, such as microbial oils, described herein are further provided. Examples of compositions comprising a microbial oil described herein include, but are not limited to, infant formulas, dietary supplements, food compositions, beverages, therapeutic drinks, nutritional drinks, cosmetic, pharmaceutical composition, etc.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

For a further understanding of the nature, objects, and advantages of the present disclosure, reference should be had to the following detailed description, read in conjunction with the following drawings, wherein like reference numerals denote like elements.

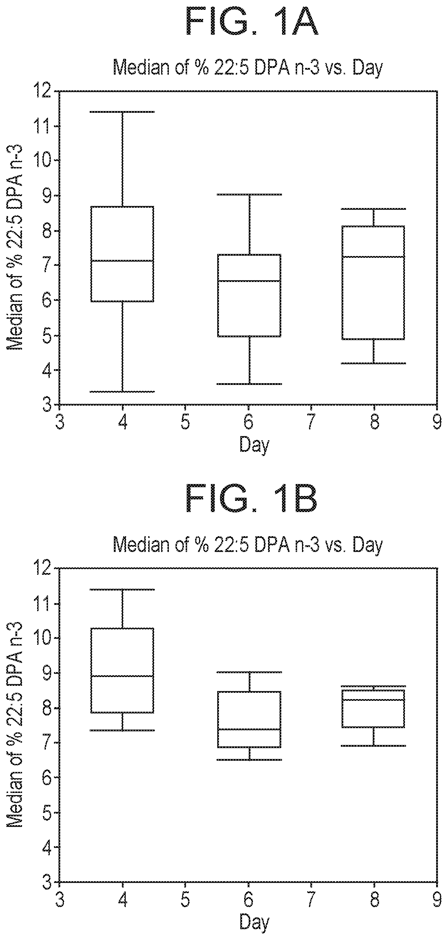

FIGS. 1A and 1B show percentage DPA(n-3) distribution of all strains tested (FIG. 1A) compared to percentage DPA(n-3) distribution of top DPA(n-3) producing strains: MK 2847, MK 2855, MK 2857, MK 2866, and MK 2867 (FIG. 1B).

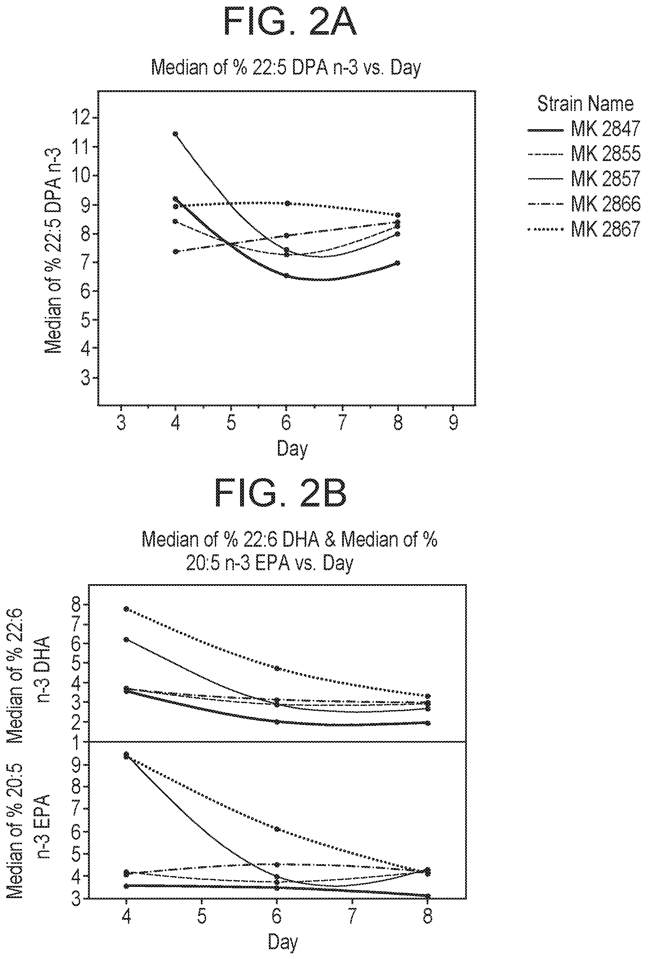

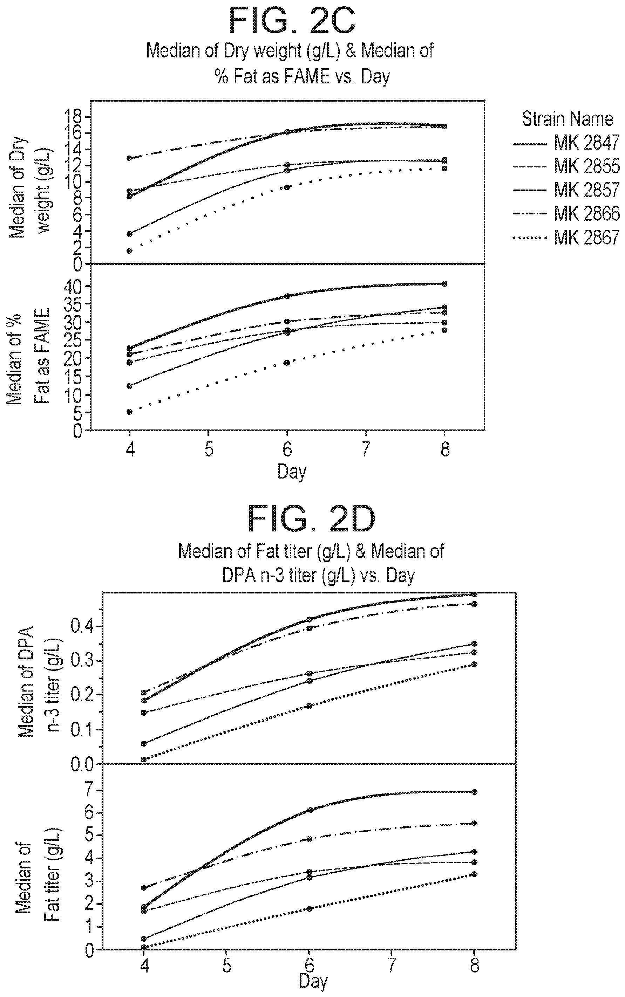

FIGS. 2A-2D show production data from selected microbial strains MK 2847, MK 2855, MK 2857, MK 2866, and MK 2867. FIG. 2A shows the median of % 22:5 DPA(n-3) over time. FIG. 2B shows the median of % 22:6 DHA (top panel) and the median of % 20:5 EPA(n-3) (bottom panel) over time. FIG. 2C shows the median of dry weight (g/L) (top panel) and the median of % Fat as FAME (bottom panel) over time. FIG. 2D shows the median of fat titer (g/L) (top panel) and the median of DPA(n-3) titer (g/L) (bottom panel) over time.

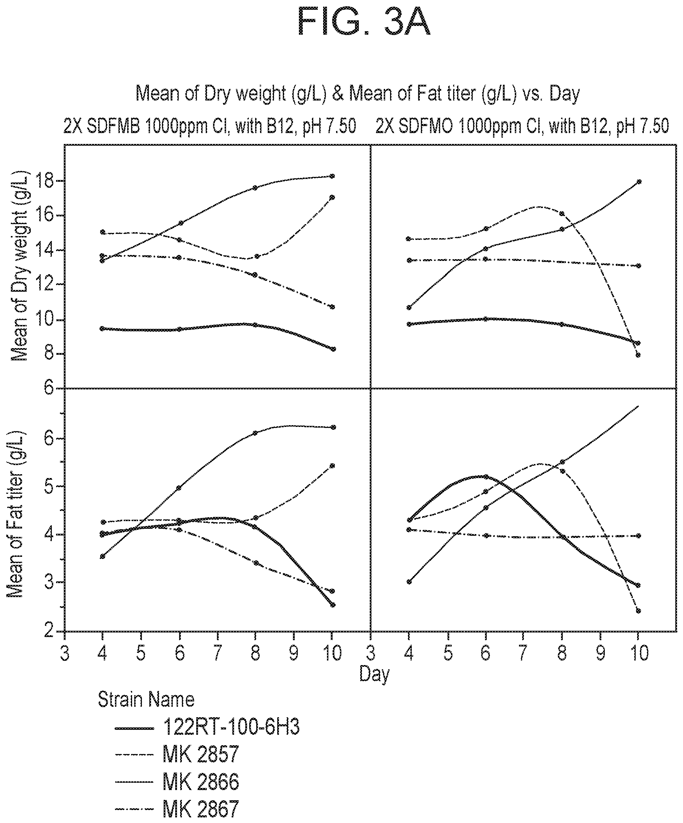

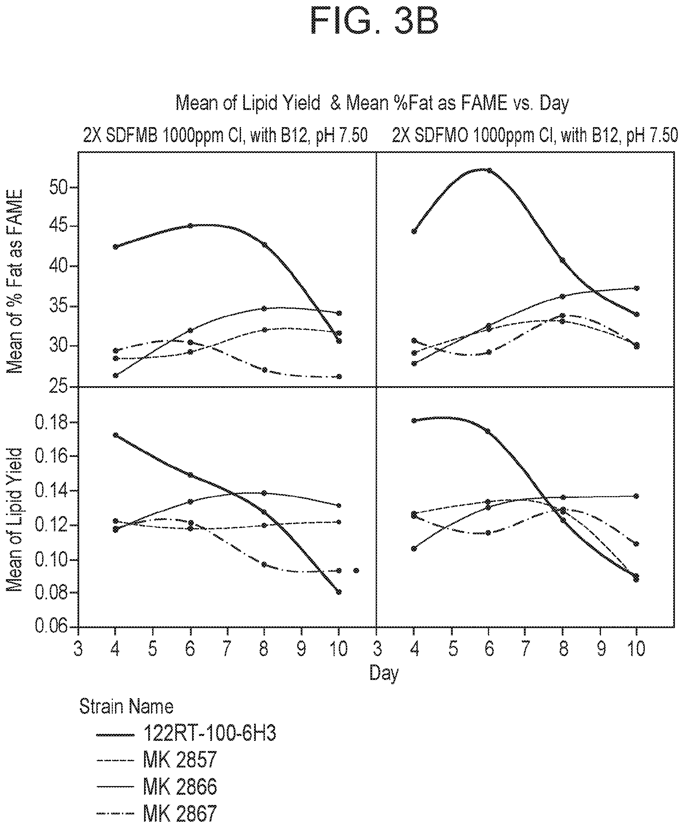

FIGS. 3A and 3B show growth (FIG. 3A) and lipid production (FIG. 3B) of selected microbial strains 122RT-100-6H3, MK 2857, MK 2866, and MK 2867 in two different media at 10% CO² at 22.5° C.

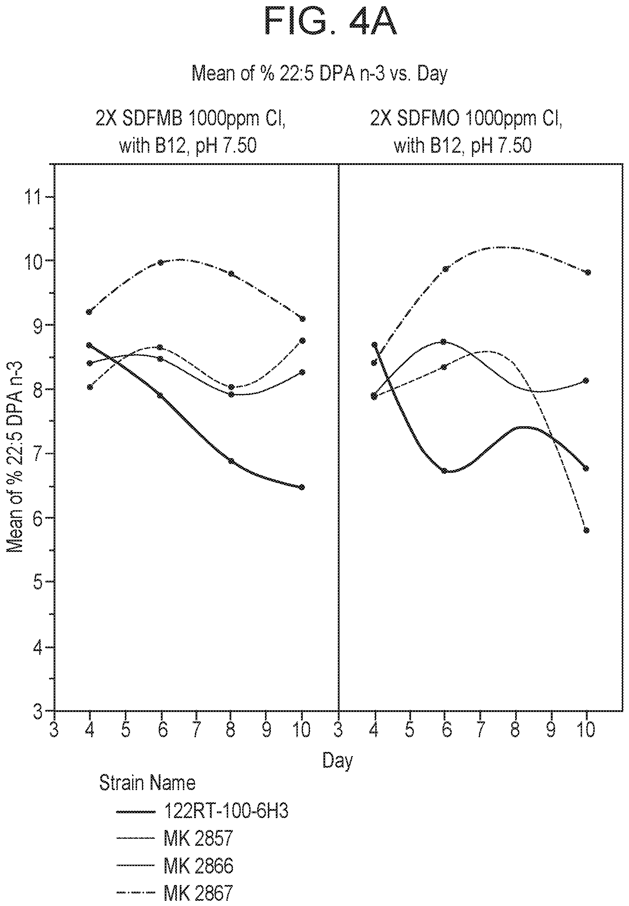

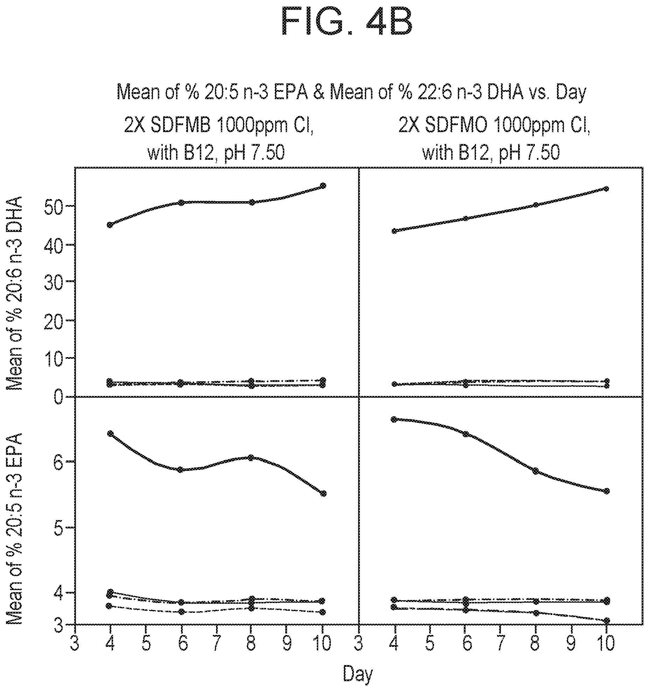

FIGS. 4A and 4B show PUFA production of selected microbial strains 122RT-100-6H3, MK 2857, MK 2866, and MK 2867 in two different media at 10% CO² at 22.5° C. FIG. 4A shows mean of % 22:5 DPA(n-3) over time. FIG. 4B shows mean of % 22:6 DHA (top panel) and mean of % 20:5 EPA(n-3) (bottom panel) over time.

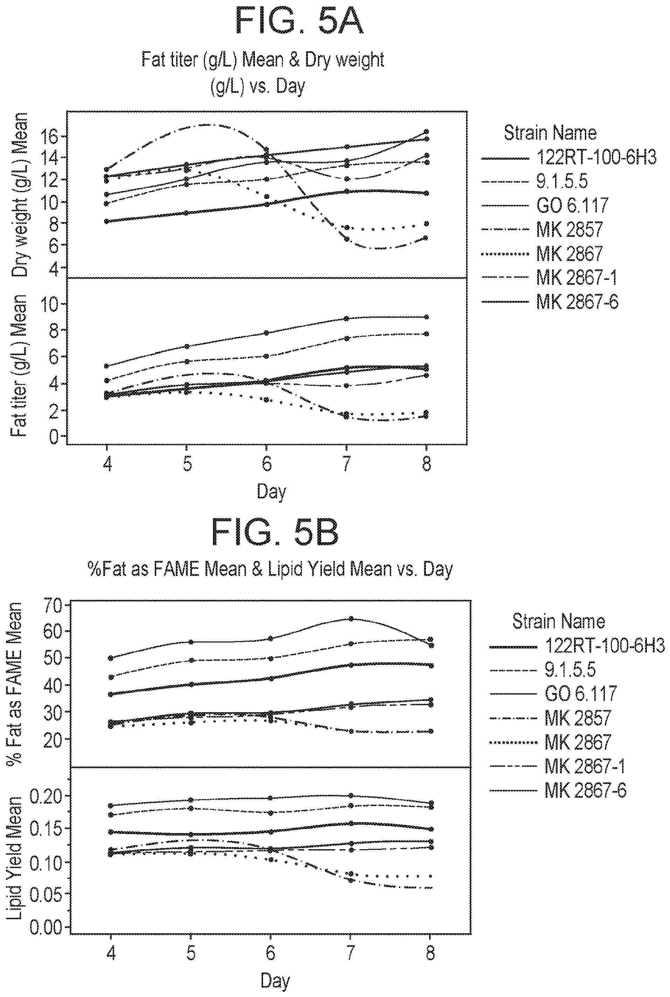

FIGS. 5A and 5B show growth (FIG. 5A) and lipid production (FIG. 5B) of selected microbial strains 122RT-100-6H3, MK 2857, MK 2867 and its clones (MK 2867-1 and MK 2867-6) compared to other production strains (9.1.5.5 and GO 6.117).

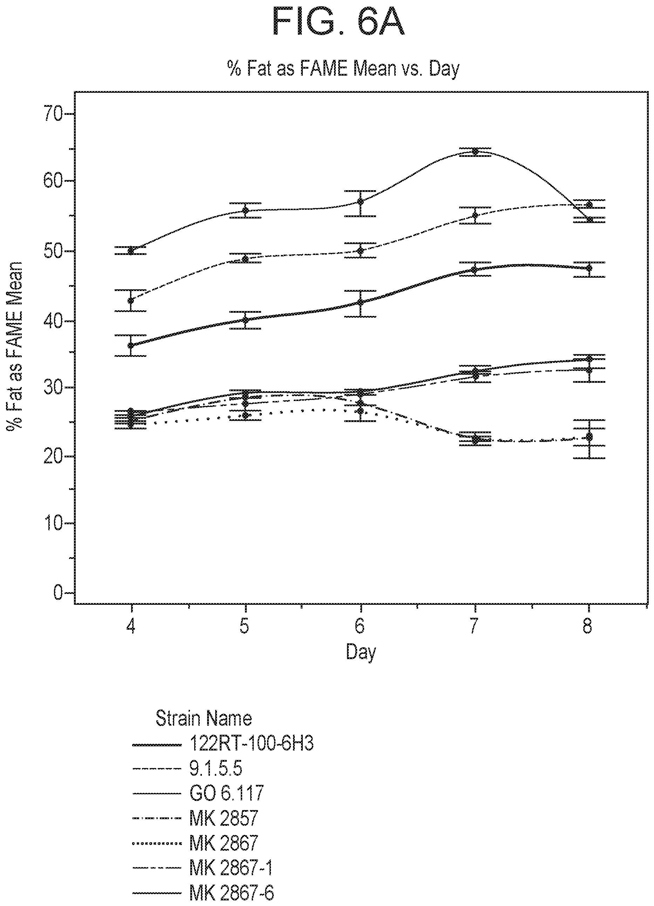

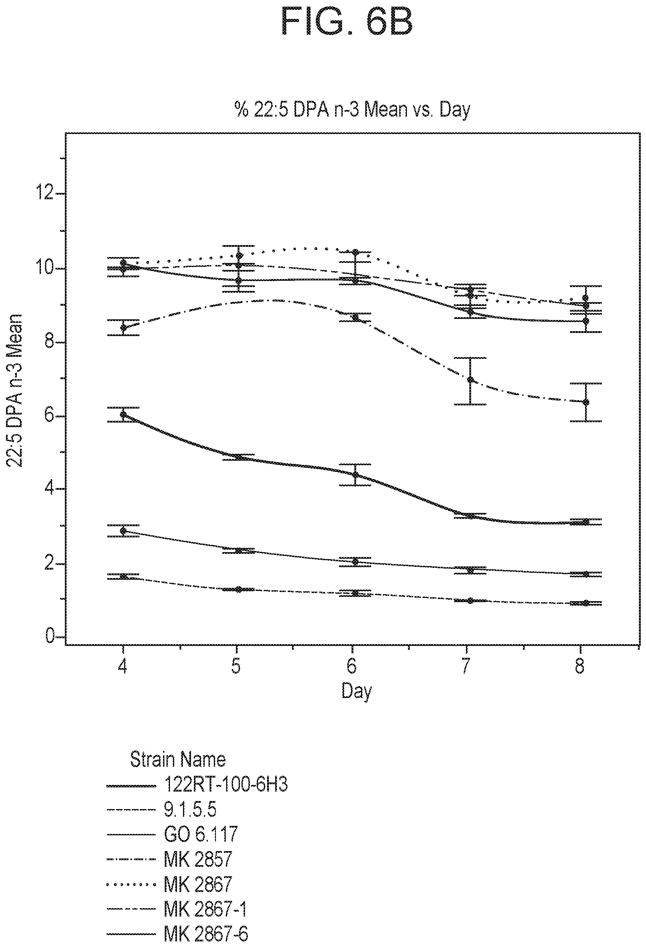

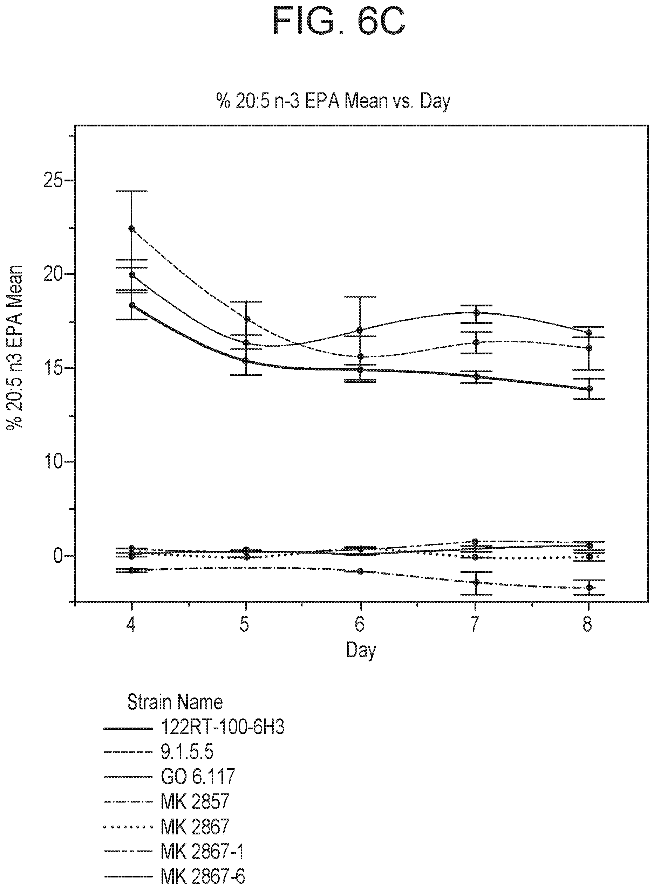

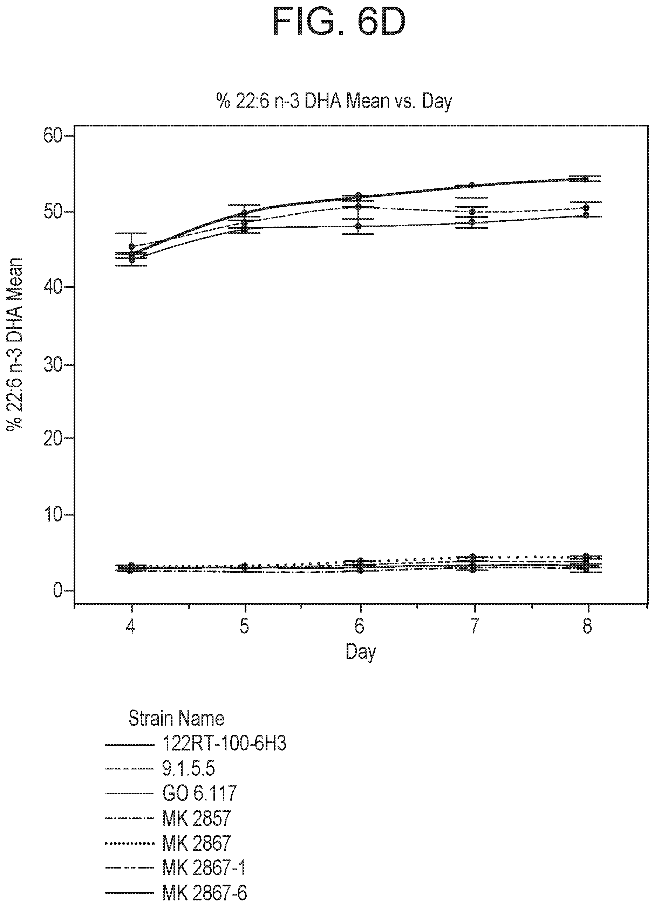

FIGS. 6A-6D show FAME and PUFA production of selected microbial strains 122RT-100-6H3, MK 2857, MK 2867 and its clones (MK 2867-1 and MK 2867-6) compared to other production strains (9.1.5.5 and GO 6.117). FIG. 6A shows mean % fat as FAME over time. FIG. 6B shows mean of % 22:5 DPA(n-3) over time. FIG. 6C shows mean of % 20:5 EPA(n-3) over time. FIG. 6D shows mean of % 22:6 DHA over time.

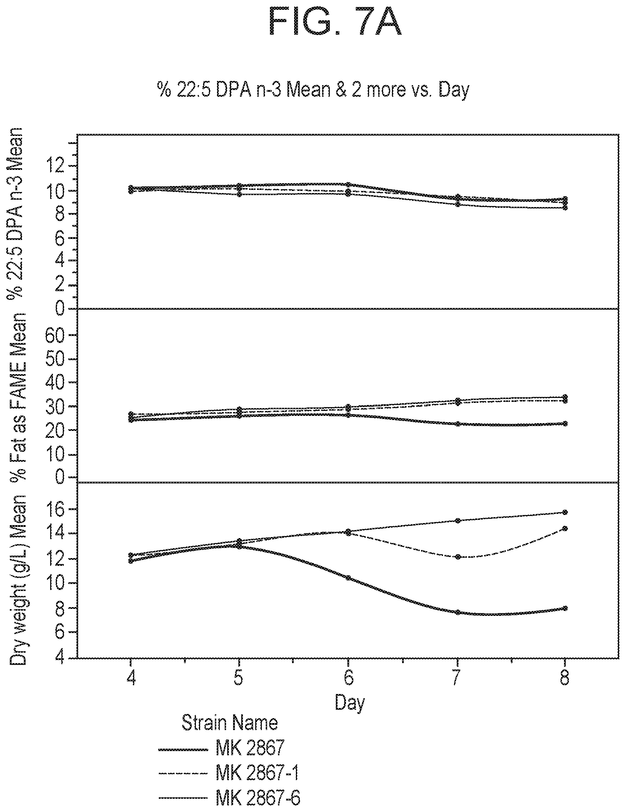

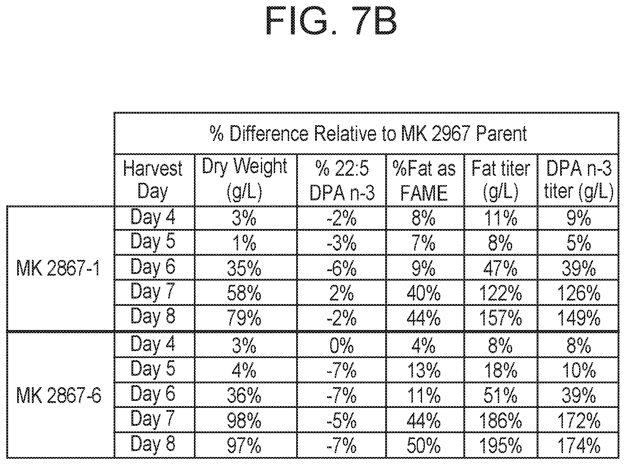

FIGS. 7A and 7B show comparison of MK 2867 clones (MK 2867-1 and MK 2867-6) to the parent strain. FIG. 7A shows the mean of % 22:5 DPA(n-3) (top panel), mean % fat as FAME (middle panel), and dry weight (g/L) over time. FIG. 7B shows the % difference of various parameters for the two clones relative to the MK 2867 parent strain.

### DETAILED DESCRIPTION

Before the subject disclosure is further described, it is to be understood that the disclosure is not limited to the particular embodiments of the disclosure described below, as variations of the particular embodiments may be made and still fall within the scope of the appended claims. It is also to be understood that the terminology employed is for the purpose of describing particular embodiments and is not intended to be limiting. Instead, the scope of the present disclosure will be established by the appended claims.

In this specification and the appended claims, the singular forms "a," "an," and "the" include plural reference unless the context clearly dictates otherwise. Unless defined otherwise, all technical and scientific terms used herein have the same meaning as commonly understood to one of ordinary skill in the art to which this disclosure belongs.

As used herein, an amount/kg/day means the amount divided by the weight of the human, animal, or infant in kilograms, per day. As used herein, an amount/L means the amount in a liter of composition (for example, infant formula) as intended for consumption by an infant, i.e., if the infant formula composition is manufactured as a dry powder or a concentrated liquid, the amount/L is measured when the dry powder or concentrated liquid has been mixed with sufficient liquid to achieve the infant formula composition intended to be consumed by an infant. When sources and amounts or ranges of the fatty acids and other ingredients are used herein, all combinations and sub-combinations and specific embodiments therein are intended to be included.

Unless otherwise specified, all weight percentages are based on weight % of the total fatty acids.

Also disclosed are methods for preparing infant formula products and infant formula compositions that are supplemented with LC-PUFAs. Infant formula is defined by United States law as a food that purports to be or is represented for special dietary use solely as a food for infants by reason of its simulation of human milk or its suitability as a complete or partial substitute for human milk.

The quality of lipids supplied to infants is of utmost importance for growth, development, and future health (Krohn et al. Macronutrient requirements for growth: fats and fatty acids. Nutrition in Pediatrics: Basic Science, Clinical, Applications. Toronto: BC Decker; 2008). According to the World Health Organization (WHO), breast-feeding is regarded as the best choice for feeding infants. The composition of human milk therefore provides some guidance for an ideal composition of breast milk substitutes. Mature human milk typically contains approximately 34% to 47% saturated fatty acids, mainly palmitic acid (17%-25%), approximately 31% to 43% monounsaturated fatty acids, approximately 12% to 26% n-6 PUFA, and approximately 0.8% to 3.6% n-3 PUFA (B. Strandvik et al. Journal of Pediatric Gastroenterology and Nutrition (2015) 61:1, 8-17). The fat in most infant formulas used today is based on a mixture of vegetable oils supplemented with long-chain polyunsaturated fatty acids (LC-PUFAs) from fish and microbial (including algae) oils, and hence has a much less complex composition than human milk fat. For example, C22:5 n-3 docosopentaenoic acid (DPA(n-3)) is the second most abundant LC-PUFA in human milk after C22:6 n-3 docosahexaenoic acid (DHA) (Lubetzky et al. Isr Med Assoc J. 2012 Jan;14(1):7-10; Nishimura et al. J Pediatr 2013 May-Jun;89(3):263-8; Martin et al. Matern Child Nutr. 2012 Jul;8(3):404-18; Zou et al. J Agric Food Chem. 2013 Jul 10; Wu et al. J Chin Med Assoc. 2010 Nov;73(11):581-8; Tijerina-Sáenz et al. Acta Paediatr. 2009 Nov;98(11):1793-8; Szlagatzys-Sidorkiewicz et al. Acta Paediatr. 2013 Aug;102(8):e353-9; Bobinski et al. Eur J Clin Nutr. 2013 Sep;67(9):966-71; Huang et al. Lipids Health Dis. 2013 Mar 6;12:27; Cruz-Hernandez et al. J Chromatogr. A. 2013 Apr 5;1284:174-9; Roy et al. Int J Food Sci Nutr. 2012 Dec;63(8):895-901; Samur et al. Lipids. 2009 May;44(5):405-13; Peng et al. Prostaglandins Leukot Essent Fatty Acids. 2009 Nov-Dec;81(5-6):325-30; Berenhauser et al. Int J Food Sci Nutr. 2012 May;63(3):318-25) but absent in infant formula. Given the growing evidence of independent and complementary effects of LC-PUFAs (S.C. Dyall Frontiers in aging neuroscience (2015), 7, 52), it is highly desirable to have DPA(n-3) present in infant formula mimicking the human milk composition.

As there is no natural vegetable, fish or microbial oil reported with a DHA:DPA(n-3):EPA ratio preferably of 1:0.5:0.3, the only option to obtain such an oil is to selectively enrich the DPA(n-3) content of natural oils or supplement the natural oils with a high DPA(n-3) containing oil. As selective enrichment processes to obtain the desired DHA:DPA(n-3):EPA ratio are technically very challenging and therefore economically not viable, others have tried to change the ratio of natural microbial oils by changing growth conditions of microbes producing the oil. For example, Li et al. (Appl Biochem Biotechnol (2017) 182, 67-81) improved the DHA:DPA(n-3) ratio from 1:0.2 to 1:0.4. However, the oil is missing EPA (DHA:DPA(n-3):EPA 1:0.4:>0.01) and still has levels of DPA(n-3) that are too low to be useful for supplementing other oils with DPA(n-3). Another approach is to screen for microorganisms which produce oils with high DPA(n-3) content (Singh and Ward; Journal of Industrial Microbiology & Biotechnology (1998) 20, 187-191) screened microorganisms and found *Pythium acanthicum* producing much higher amounts of DPA(n-3) than DHA but still high amounts of EPA (DPA(n-3):EPA ratio of 1:0.95).

In one embodiment, the invention provides a solution to obtain a composition, including but not limited to an infant formula composition, with a preferred DHA:DPA(n-3):EPA ratio of 1:0.5:0.3 by providing natural DPA(n-3) enriched oils from microbial sources that produce an oil that contain DHA, DPA, and EPA, with a DHA:DPA(n-3):EPA ratio of <0.7: 1:<0.7 (that is, they contain non-zero levels of DHA and EPA). These oils avoid expensive enrichment processes and can be mixed with other natural oils to obtain an infant formula mimicking human milk's LC-PUFA composition.

As noted above, it has been suggested that LC-PUFAs be included in infant formula in amounts that match or closely mimic the LC-PUFA profile found in human breast milk. The formulas of the present invention are based on the recognition that infant formula supplemented with DPA(n-3) to match or closely mimic the amount of DPA(n-3) present in human breast milk provides significant advantages over currently available infant formulas comprising LC-PUFAs.

Since the infant formulas of the invention comprise multiple components, including LC-PUFAs, it will be appreciated that the different embodiments of the infant formulas can be described by reference to one or more of the components, or ratios of such components. For example, the amount of DPA(n-3) in the composition can be expressed as ratio with DHA and/or EPA or as a concentration of DPA(n-3) contained in the formula, e.g., in a liter of the formula as intended for consumption by an infant. The infant formulas can also be described by using the amount of DPA(n-3) in the composition based on the DPA(n-3) plasma levels that are desired.

Accordingly, in an embodiment, the infant formula composition comprises DPA(n-3) in an amount similar to that found in human breast milk. It has been shown that DPA(n-3) content in human breast milk can vary over time (e.g., colostrum vs. mature breast milk) and can also vary depending on the individual and/or environment. Thus, the amount of DPA(n-3) included in an infant formula composition of the invention may also vary. In some embodiments, the infant formula composition comprises 0.06 to 0.75% DPA(n-3) as a percentage of total lipid content in the formula. In some embodiments, the infant formula composition comprises 0.14 to 0.29% DPA(n-3) as a percentage of total lipid content in the formula.

The ratio of DPA(n-3):DHA in the composition is greater than 0.2:1. In some embodiments, the ratio of DPA(n-3):DHA in the composition is greater than about 0.3:1, greater than about 0.4:1, greater than about 0.5:1, greater than about 0.6:1, greater than about 0.7:1, or greater than about 0.8:1.

In some embodiments, the DPA(n-3):DHA ratio in the infant formula composition is from about 0.2:1 to about 1:1, from about 0.3:1 to about 1:1, from about 0.4:1 to about 1:1, from about 0.5:1 to about 1:1, from about 0.6:1 to about 1:1, from about 0.7:1 to about 1:1, from about 0.2 to about 0.9, from about 0.2 to about 0.8, from about 0.3 to about 0.8, about 0.2:1, about 0.3:1, about 0.4:1, about 0.5:1, about 0.6:1, about 0.7:1, about 0.8:1, about 0.9:1, or about 1:1.

In some embodiments, the DHA:DPA(n-3):EPA ratio in the infant formula composition is 1:0.5:0.3.

The long chain polyunsaturated fatty acids in the composition are supplied in a source oil, wherein the source oil comprises DPA(n-3) in an amount of at least about 7% by weight. In certain embodiments, the source oil comprises long chain n-3 fatty acids comprising at least about 8% DPA(n-3) by weight, at least about 9% DPA(n-3) by weight, at least about 10% DPA(n-3) by weight, at least about 15% DPA(n-3) by weight, at least about 20% DPA(n-3) by weight, at least about 25% DPA(n-3) by weight, at least about 30% DPA(n-3) by weight, or at least about 35% DPA(n-3) by weight. In some embodiments, the microbial oil comprises between about 7% to about 50% DPA(n-3) by weight, between about 7% to about 35% DPA(n-3) by weight, between about 7% to about 20% DPA(n-3) by weight, between about 7% to about 15% DPA(n-3) by weight, between about 7% to about 14% DPA(n-3) by weight, between about 7% to about 13% DPA(n-3) by weight, between about 7% to about 12% DPA(n-3) by weight, between about 7% to about 11% DPA(n-3) by weight, between about 7% to about 10% DPA(n-3) by weight, or between about 7% to about 9% DPA(n-3) by weight. In some embodiments, the microbial oil comprises between about 8% to about 15% DPA(n-3) by weight, between about 8% to about 14% DPA(n-3) by weight, between about 8% to about 13% DPA(n-3) by weight, between about 8% to about 12% DPA(n-3) by weight, between about 8% to about 11% DPA(n-3) by weight, or between about 8% to about 10% DPA(n-3) by weight.

In some embodiments, the source oil comprises less than about 5% DHA by weight, less than about 4.5% DHA by weight, less than about 4% DHA by weight, less than about 3.5% DHA by weight, less than about 3% DHA by weight, less than about 2.5% DHA by weight, or less than about 2% DHA by weight. In some embodiments, the microbial oil comprises between about 1% to about 5% DHA by weight, between about 1% to about 4% DHA by weight, or between about 2% to about 4% DHA by weight.

In certain embodiments, the DPA(n-3):DHA ratio is from about 0.2:1 to about 1:1, from about 0.3:1 to about 1:1, from about 0.4:1 to about 1:1, from about 0.5:1 to about 1:1, from about 0.6:1 to about 1:1, from about 0.7:1 to about 1:1, from about 0.2 to about 0.9, from about 0.2 to about 0.8, from about 0.3 to about 0.8, about 0.2:1, about 0.3:1, about 0.4:1, about 0.5:1, about 0.6:1, about 0.7:1, about 0.8:1, about 0.9:1, or about 1:1.

In some embodiments, the ratio of DHA:DPA(n-3):EPA in the source oil is <0.7:1:<0.7.

In certain embodiments, the infant formula composition comprises at least 20 mg/L of DHA or at least 40 mg/L DHA. In other embodiments, the infant formula composition comprises from about 40 to about 140 mg/L of DHA, from about 20 to about 200 mg/L of DHA. In other embodiments, the composition comprises less than about 140 mg/L of DHA or less than about 200 mg/L of DHA.

In certain embodiments, the infant formula composition comprises at least about 7 mg/L of DPA(n-6), at least about 13 mg/L of DPA(n-6), at least about 26 mg/L of DPA(n-6), at least about 40 mg/L of DPA(n-6), at least about 53 mg/L of DPA(n-6), or at least about 66 mg/L of DPA(n-6), at least about 80 mg/L of DPA(n-6), at least about 100 mg/L of DPA(n-6), at least about 120 mg/L of DPA(n-6). In other embodiments, the composition comprises less than about 240 mg/L of DPA(n-6), less than about 220 mg/L of DPA(n-6), or less than about 200 mg/L of DPA(n-6).

In certain other embodiments, the infant formula composition comprises eicosapentaenoic acid (EPA) in an amount less than about 3 mg/L. In certain other embodiments, the infant formula composition comprises EPA in an amount less than about 60 mg/L. In other embodiments, the infant formula composition comprises from about 15 mg/L to about 30 mg/L EPA. In some embodiments, the EPA:DHA ratio is provided in a ratio of up to 1:1.

In certain other embodiments, the infant formula composition is formulated to provide at least about 5 mg/kg/day DPA(n-3) when administered to an infant. In certain embodiments, the infant formula composition is formulated to provide from about 5 mg/kg/day DPA(n-3) to about 45 mg/kg/day DPA(n-3) when administered to an infant.

The invention also provides a method of preparing a composition, comprising combining nutritional components and long chain n-3 fatty acids; wherein the long chain n-3 fatty acids comprise DPA(n-3) and DHA; wherein the ratio of DPA(n-3):DHA is from about 0.2:1 to about 1:1. Infant formula compositions prepared by this method are also included in the invention.

In some embodiments, a method of preparing a composition is provided comprising combining nutritional components and an oil blend, wherein the oil blend comprises a first oil comprising one or more of a plant oil, a fish oil, or a microbial oil, and a second oil, wherein the second oil is a microbial oil having a DHA:DPA(n-3) ratio of <0.7:1, preferably a microbial oil having a DHA:DPA(n-3):EPA ratio of <0.7: 1:<0.7. Compositions prepared by this method are also included in the invention.

The DPA, DHA, and other PUFAs referred to herein, such as ARA and EPA, can be in any of the common forms found in natural lipids including but not limited to triacylglycerols, diacylglycerols, monoacylglycerols, phospholipids, free fatty acids, esterified fatty acids, or in natural or synthetic derivative forms of these fatty acids (e.g., calcium salts of fatty acids, ethyl esters, etc). Reference to an oil comprising a PUFA, as used in the present invention, can refer to either an oil comprising only a single PUFA such as DHA or an oil comprising a mixture of two or more PUFAs such as DHA and EPA, or DHA and DPA.

A preferred source of an oil comprising at least one PUFA, in the compositions and methods of the present invention, includes a microbial source. Microbial sources and methods for growing microorganisms comprising nutrients and/or PUFAs are known in the art (Industrial Microbiology and Biotechnology, 2nd edition, 1999, American Society for Microbiology). Preferably, the microorganisms are cultured in a fermentation medium in a fermenter. The methods and compositions of the present invention are applicable to any industrial microorganism that produces any kind of nutrient or desired component such as, for example algae, protists, bacteria and fungi (including yeast).

Microbial sources can include a microorganism such as an algae, bacteria, fungi and/or protist. Preferred organisms include those selected from the group consisting of golden algae (such as microorganisms of the kingdom Stramenopiles), green algae, diatoms, dinoflagellates (such as microorganisms of the order Dinophyceae including members of the genus *Crypthecodinium* such as, for example, *Crypthecodinium cohnii*), yeast, and fungi of the genera *Mucor* and *Mortierella,* including but not limited to *Mortierella alpina* and *Mortierella* sect. *schmuckeri.* Members of the microbial group Stramenopiles include microalgae and algae-like microorganisms, including the following groups of microorganisms: Hamatores, Proteromonads, Opalines, Develpayella, Diplophrys, Labrinthulids, Thraustochytrids, Biosecids, Oomycetes, Hypochytridiomycetes, Commation, Reticulosphaera, Pelagomonas, Pelagococcus, Ollicola, Aureococcus, Parmales, Diatoms, Xanthophytes, Phaeophytes (brown algae), Eustigmatophytes, Raphidophytes, Synurids, Axodines (including Rhizochromulinaales, Pedinellales, Dictyochales), Chrysomeridales, Sarcinochrysidales, Hydrurales, Hibberdiales, and Chromulinales. The Thraustochytrids include the genera *Schizochytrium* (species include *aggregatum, limnaceum, mangrovei, minutum, octosporum*), *Thraustochytrium* (species include *arudimentale, aureum, benthicola, globosum, kinnei, motivum, multirudimentale, pachydermum, proliferum, roseum, striatum*), *Ulkenia** (species include *amoeboidea, kerguelensis, minuta, profunda, radiate, sailens, sarkariana, schizochytrops, visurgensis, yorkensis*), *Aplanochytrium* (species include *haliotidis, kerguelensis, profunda, stocchinoi*), *Japonochytrium* (species include *marinum*), *Althornia* (species include *crouchii*), and *Elina* (species include *marisalba, sinorifica*)*.* Since there is some disagreement among experts as to whether *Ulkenia* is a separate genus from the genus *Thraustochytrium,* for the purposes of this application, the genus *Thraustochytrium* will include *Ulkenia.* The Labyrinthulids include the genera *Labyrinthula* (species include *algeriensis, coenocystis, chattonii, macrocystis, macrocystis atlantica, macrocystis, marina, minuta, roscoffensis, valkanovii, vitellina, vitellina pacifica, vitellina, zopfi), Labyrinthomyxa* (species include *marina*)*, Labyrinthuloides* (species include *haliotidis, yorkensis*), *Diplophrys* (species include *archeri*)*, Pyrrhosorus** (species include *marinus*), *Sorodiplophrys** (species include *stercorea*)*, Chlamydomyxa** (species include *labyrinthuloides, montana*). (* = there is no current general consensus on the exact taxonomic placement of these genera).

It has been discovered herein that protists of the Ichthyosporea class are excellent sources for DPA(n-3). Accordingly, another preferred source of an oil comprising at least one PUFA, in the compositions and methods of the present invention, includes a protist of the Ichthyosporea class. Examples of protists within the Ichthyosporea class include but are not limited to Ichthyophonida such as *Creolimax fragrantissima, Sphaeroforma, Anurofeca richardsi, Psoropermium, Caullerya mesnili, Pirum gemmata, Abeoforma whisleri, Amoebidium, Paramoebidium, Eccrinales,* and *Icthyophonus,* and *Dermocystida* such as *Rhinosporidium seeberi, Dermocystidium percae, Sphaerothecum destruens, Amphibiothecum penneri, Amphibiocystidium ranae,* and *Dermocystidium salmonis. Sphaeroforma arctica* is particularly preferred as a source of an oil comprising at least one PUFA.

While processes of the present invention can be used to produce forms of PUFAs that can be produced in a wide variety of microorganisms, for the sake of brevity, convenience and illustration, this detailed description of the invention will discuss processes for growing microorganisms which are capable of producing lipids comprising omega-3 and/or omega-6 polyunsaturated fatty acids, in particular microorganisms that are capable of producing DHA (or closely related compounds such as DPA, EPA or ARA). More preferably, the microorganisms are selected from the group consisting of microorganisms having the identifying characteristics of ATCC number 20888, ATCC number 20889, ATCC number 20890, ATCC number 20891 and ATCC number 20892. Also preferred are strains of Mortierella schmuckeri (e.g., including microorganisms having the identifying characteristics of ATCC 74371) and Mortierella alpina (e.g., including microorganisms having the identifying characteristics of ATCC 42430). Also preferred are strains of Crypthecodinium cohnii, including microorganisms having the identifying characteristics of ATCC Nos. 30021, 30334-30348, 30541-30543, 30555-30557, 30571, 30572, 30772-30775, 30812, 40750, 50050-50060, and 50297-50300. Also preferred are mutant strains derived from any of the foregoing, and mixtures thereof. Oleaginous microorganisms are also preferred. As used herein, "oleaginous microorganisms" are defined as microorganisms capable of accumulating greater than 20% of the weight of their cells in the form of lipids. Genetically modified microorganisms that produce PUFAs are also suitable for the present invention. These can include naturally PUFA-producing microorganisms that have been genetically modified as well as microorganisms that do not naturally produce PUFAs but that have been genetically modified to do so.

Suitable organisms may be obtained from a number of available sources, including by collection from the natural environment. For example, the American Type Culture Collection currently lists many publicly available strains of microorganisms identified above. As used herein, any organism, or any specific type of organism, includes wild strains, mutants, or recombinant types. Growth conditions in which to culture or grow these organisms are known in the art, and appropriate growth conditions for at least some of these organisms are disclosed in, for example, U.S. Patent No. 5,130,242, U.S. Patent No. 5,407,957, U.S. Patent No. 5,397,591, U.S. Patent No. 5,492,938, and U.S. Patent No. 5,711,983, all of which are incorporated herein by reference in their entirety.

Another preferred source of an oil comprising at least one PUFA, in the compositions and methods of the present invention includes an animal source. Thus, in some embodiments, the oil comprising at least one PUFA is an aquatic animal oil. Examples of animal sources include aquatic animals (e.g., fish, marine mammals, and crustaceans such as krill and other euphausids) and lipids extracted from animal tissues (e.g., brain, liver, eyes, etc.) and animal products such as eggs or milk.

The invention further provides compositions comprising blends of microbial oils and blends of microbial and plant oils.

Nutritional components of infant formulas are known in the art and one knowledgeable in the art would be able to adjust formula compositions to include PUFA levels and ratios of the instant invention. For example, an infant formula typically contains a protein component comprising from about 6 to about 25% of the total caloric content of the infant formula; a carbohydrate component comprising from about 35 to about 50% of the total caloric content of the infant formula; and a lipid component comprising from about 30 to about 50% of the total caloric content of the infant formula. These ranges are provided as examples only and are not intended to be limiting.

Examples of suitable fat sources typically include high oleic safflower oil, soy oil, fractionated coconut oil (medium chain triglycerides, MCT oil), high oleic sunflower oil, corn oil, canola oil, coconut, palm and palm kernel oils, marine oil, cottonseed oil, walnut oil, wheat germ oil, sesame oil, cod liver oil, and peanut oil. Any single fat listed above, or any combination thereof, as appropriate may be utilized. Other suitable fats will be readily apparent to those skilled in the art.

Additional components of infant formula typically include, for example, protein, carbohydrates, vitamins and minerals. Examples of suitable protein sources for an infant typically include casein, whey, condensed skim milk, nonfat milk, soy, pea, rice, corn, hydrolyzed protein, free amino acids, protein sources which contain calcium in a colloidal suspension with the protein. Any single protein listed above, or any combination thereof, as appropriate may be utilized. Other suitable proteins will be readily apparent to those skilled in the art.

A third component of infant formula is a source of carbohydrates. Carbohydrates are a major source of readily available energy that the infant needs for growth and that protects the infant from tissue catabolism. In human milk and most standard milk-based infant formulas, the carbohydrate is lactose. The carbohydrates that may be used in the infant formula can vary widely. Examples of carbohydrates suitable for infants typically include hydrolyzed cornstarch, maltodextrin, glucose polymers, sucrose, lactose, corn syrup, corn syrup solids, rice syrup, glucose, fructose, high fructose corn syrup and indigestible oligosaccharides such as fructooligosaccharides (FOS). Any single carbohydrate listed above, or any combination thereof, as appropriate may be utilized. Other suitable carbohydrates will be readily apparent to those skilled in the art.

The infant formula of the present invention typically includes supplemented vitamins and minerals. Examples of vitamins and minerals that may be added to the infant formula of the instant invention typically include vitamin A, vitamin B₁, vitamin B₂, vitamin B₆, vitamin B₁₂, vitamin C, vitamin D, vitamin K, vitamin E₅ biotin, folic acid, pantothenic acid, niacin, m-inositol, calcium, phosphorus, magnesium, zinc, manganese, copper, sodium, potassium, chloride, iron and selenium. The additional nutrients chromium, molybdenum, iodine, taurine, carnitine and choline may also be included.

The infant formulas of the present invention may be prepared as any product form suitable for use in infants, including reconstitutable powders, ready-to-feed liquids, and dilutable liquid concentrates, which product forms are all well known in the nutritional formula art. As used in the present application, the amounts of components present in infant formula compositions refer to the amounts when the formula is ready for consumption by the infant. It is to be understood that in the case of a reconstitutable powder or dilutable liquid concentrate, the component amounts will be adjusted such that when the infant formula composition is reconstituted or diluted the amounts are as described herein. Thus, for example, reference to an infant formula composition that is to be diluted by, for example, addition of one part water for one part infant formula, wherein the infant formula composition has a given component concentration, when ready for consumption, is intended to cover an infant formula composition having a concentration of the component of twice the given amount, before it is made ready for consumption by the addition of water. Methods to prepare infant formulas are known to those skilled in the art. For example, the PUFA-containing oils can be added directly to a liquid formula composition at a suitable point in the manufacturing process.

The infant formula according to the present invention can optionally be sterilized and subsequently used on a ready-to-feed basis or can be stored as a concentrate. The concentrate can be prepared by spray drying the liquid formula prepared as above, and the formula can be reconstituted by rehydrating the concentrate. The infant formula concentrate is a stable liquid and has a suitable shelf life.

In another embodiment, the oils are microencapsulated prior to the addition into a formula composition. The choice of coating for the microencapsulation of the PUFAs is determined by its lack of toxicity, desired particle size, and stability under the processing conditions for such compositions, particularly sterilization. Any conventionally acceptable substantially oxygen-impermeable coating can be used in the present invention. Such conventional microencapsulating methods and coating materials are well within the purview of one skilled in the art, and the specific microencapsulating method and coating are not peculiar to the present invention. Some of these methods include spray drying such as where the PUFAs are emulsified into a solution of a polymer, and spray-dried to make fine particles. Particles of about 250 µm are suitable for inclusion in the infant formulas according to the present invention. When the PUFAs are incorporated into a meltable fat or wax, the process is called spray-chilling, since the emulsion need only be chilled below its melting point to form particles. Another encapsulation process that can be used to encapsulate the PUFAs is coacervation. Other suitable techniques include interfacial polymerization, hot melt encapsulation, phase separation encapsulation (solvent removal and solvent evaporation), spontaneous emulsion, solvent evaporation microencapsulation, solvent removal microencapsulation, coacervation, and low temperature microsphere formation and phase inversion nanoencapsulation (PIN). In general, the microencapsulated PUFAs form a free-flowing powder which is suitable for addition into powdered embodiments of the compositions. These and other encapsulation techniques and encapsulated oils are described in United States Provisional Patent Application Ser. No. 60/805,590, which is incorporated by reference herein in its entirety.

For powder embodiments of the present invention, the above-described methods of use further comprise reconstitution of the powder with a suitable aqueous liquid, preferably water. Such dilution may be in an amount sufficient to provide an LC-PUFA fortified infant formula having the characteristics described in detail herein. For powder embodiments of the present invention, such powders are typically in the form of flowable or substantially flowable particulate compositions, or at least particulate compositions that can be easily scooped and measured with a spoon or similar other device, wherein the compositions can easily be reconstituted by the intended user with a suitable aqueous fluid, typically water, to form a liquid infant formula. In this context, "immediate" use generally means within about 48 hours, most typically within about 24 hours, preferably right after reconstitution. These powder embodiments include spray dried, agglomerated, dry mixed or other known or otherwise effective particulate form. The quantity of a nutritional powder required to produce a volume suitable for one serving can vary.

The nutritional formulas of the present invention may be packaged and sealed in single or multi-use containers, and then stored under ambient conditions for up to about 36 months or longer, more typically from about 12 to about 24 months. For multi-use containers, these packages can be opened and then covered for repeated use by the ultimate user, provided that the covered package is then stored under ambient conditions (e.g., avoid extreme temperatures) and the contents used within about one month or so.

Premature infants require additional nutrients to support their growth and are at risk for the diseases related to prematurity. Preterm infants are commonly fed either a commercial infant formula designed specifically for these infants or their own mother's milk. Another means of feeding a preterm infant is to supplement preterm milk, banked term milk, other suitable milk, or infant formula with a milk or formula fortifier. Such supplemented milk or formula can more adequately provide levels of several nutrients to meet the needs of these infants. Another invention of this application provides a premature infant nutrition fortifier composition comprising LC-PUFAs. In particular, the premature infant nutrition fortifier composition comprises DPA(n-3) and in other embodiments, can comprise DHA, EPA, and/or ARA. The fortifier composition is generally a powder or oil which can optionally supplements level of protein, fat, vitamins and minerals. The fortifier compositions are formulated to provide the amounts and ratios of LC-PUFAs as described for infant formula compositions above, when added to milk or formula. For example, a fortified milk or formula composition can comprise a threshold amount of DPA(n-3), can have a DHA:DPA(n-3) ratio that is from about 1:0.3 to 1:0.8, or have other limitations from the various inventions described herein. The fortifier compositions can be the sole source of one or more of the DPA(n-3), DHA, EPA, and/or ARA LC-PUFAs in the resulting fortified milk or formula or can supplement amounts of LC PUFAs in the unfortified milk or formula.

As used in the present invention, a microbial oil is disclosed comprising DPA(n-3) in an amount of at least about 7% by weight, wherein the DPA(n-3):DHA ratio is greater than 0.2.

In some embodiments, the microbial oil comprises DPA(n-3) in an amount between about 7 to about 11% by weight or about 8 to about 10% by weight.

In some embodiments, the DPA(n-3):DHA ratio is from about 0.2:1 to about 1:1, from about 0.3:1 to about 1:1, from about 0.4:1 to about 1:1, from about 0.5:1 to about 1:1, from about 0.6:1 to about 1:1, from about 0.7:1 to about 1:1, from about 0.2 to about 0.9, from about 0.2 to about 0.8, from about 0.3 to about 0.8, about 0.2:1, about 0.3:1, about 0.4:1, about 0.5:1, about 0.6:1, about 0.7:1, about 0.8:1, about 0.9:1, or about 1:1.

In some embodiments, the microbial oil comprises long chain n-3 fatty acids comprising at least about 7% DPA(n-3) by weight. In some embodiments, the microbial oil comprises long chain n-3 fatty acids comprising at least about 8% DPA(n-3) by weight, at least about 9% DPA(n-3) by weight, at least about 10% DPA(n-3) by weight, at least about 15% DPA(n-3) by weight, at least about 20% DPA(n-3) by weight, at least about 25% DPA(n-3) by weight, at least about 30% DPA(n-3) by weight, or at least about 35% DPA(n-3) by weight. In some embodiments, the microbial oil comprises between about 7% to about 50% DPA(n-3) by weight, between about 7% to about 35% DPA(n-3) by weight, between about 7% to about 20% DPA(n-3) by weight, between about 7% to about 15% DPA(n-3) by weight, between about 7% to about 14% DPA(n-3) by weight, between about 7% to about 13% DPA(n-3) by weight, between about 7% to about 12% DPA(n-3) by weight, between about 7% to about 11% DPA(n-3) by weight, between about 7% to about 10% DPA(n-3) by weight, or between about 7% to about 9% DPA(n-3) by weight. In some embodiments, the microbial oil comprises between about 8% to about 15% DPA(n-3) by weight, between about 8% to about 14% DPA(n-3) by weight, between about 8% to about 13% DPA(n-3) by weight, between about 8% to about 12% DPA(n-3) by weight, between about 8% to about 11% DPA(n-3) by weight, or between about 8% to about 10% DPA(n-3) by weight.

In some embodiments, the microbial oil comprises less than about 5% DHA by weight, less than about 4.5% DHA by weight, less than about 4% DHA by weight, less than about 3.5% DHA by weight, less than about 3% DHA by weight, less than about 2.5% DHA by weight, or less than about 2% DHA by weight. In some embodiments, the microbial oil comprises between about 1% to about 5% DHA by weight, between about 1% to about 4% DHA by weight, or between about 2% to about 4% DHA by weight.

In some embodiments, the microbial oil comprises less than about 5% EPA by weight, less than about 4.5% EPA by weight, less than about 4% EPA by weight, less than about 3.5% EPA by weight, less than about 3% EPA by weight, less than about 2.5% EPA by weight, or less than about 2% EPA by weight. In some embodiments, the microbial oil comprises between about 1% to about 5% EPA by weight, between about 2% to about 5% EPA by weight, or between about 3% to about 5% EPA by weight.

In some embodiments, the microbial oil comprises a total fat content greater than about 20% by weight, greater than about 21% by weight, greater than about 22% by weight, greater than about 23% by weight, greater than about 24% by weight, greater than about 25% by weight, greater than about 26% by weight, greater than about 27% by weight, greater than about 28% by weight, greater than about 29% by weight, greater than about 30% by weight, greater than about 31% by weight, greater than about 32% by weight, greater than about 33% by weight, greater than about 34% by weight, or greater than about 35% by weight. In some embodiments, the microbial oil comprises a total fat content between about 20 to about 40% by weight, between about 20 to about 35% by weight, between about 25 to about 40% by weight, or between about 25 to about 35% by weight.

In some embodiments, the microbial oil is produced by one or more microorganisms described herein. Preferred microorganisms include those selected from the group consisting of golden algae (such as microorganisms of the kingdom Stramenopiles), green algae, diatoms, dinoflagellates (such as microorganisms of the order Dinophyceae including members of the genus Crypthecodinium such as, for example, Crypthecodinium cohnii), yeast, and fungi of the genera Mucor and Mortierella, including but not limited to Mortierella alpina and Mortierella sect, schmuckeri. Members of the microbial group Stramenopiles include microalgae and algae-like microorganisms, including the following groups of microorganisms: Hamatores, Proteromonads, Opalines, Develpayella, Diplophrys, Labrinthulids, Thraustochytrids, Biosecids, Oomycetes, Hypochytridiomycetes, Commation, Reticulosphaera, Pelagomonas, Pelagococcus, Ollicola, Aureococcus, Parmales, Diatoms, Xanthophytes, Phaeophytes (brown algae), Eustigmatophytes, Raphidophytes, Synurids, Axodines (including Rhizochromulinaales, Pedinellales, Dictyochales), Chrysomeridales, Sarcinochrysidales, Hydrurales, Hibberdiales, and Chromulinales. The Thraustochytrids include the genera Schizochytrium (species include aggregation, limnaceum, mangrovei, minutum, octosporum), Thraustochytrium (species include arudimentale, aureum, benthicola, globosum, kinnei, motivum, multirudimentale, pachydermum, proliferum, roseum, striatum), Ulkenia* (species include amoeboidea, kerguelensis, minuta, profunda, radiate, sailens, sarkariana, schizochytrops, visurgensis, yorkensis), Aplanochytrium (species include haliotidis, kerguelensis, profunda, stocchino 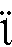 ), Japonochytrium (species include marinum), Althornia (species include crouchi 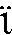 ), and Elina (species include marisalba, sinorifica). Since there is some disagreement among experts as to whether Ulkenia is a separate genus from the genus Thraustochytrium, for the purposes of this application, the genus Thraustochytrium will include Ulkenia. The Labrinthulids include the genera Labyrinihula (species include algeriensis, coenocystis, chattonii, macrocystis, macrocystis atlantica, macrocystis, marina, minuta, roscoffensis, valkanovii, vitellina, vitellina paciβca, vitellina, zopβ), Labyrinthomyxa (species include marina), Labyrinthuloides (species include haliotidis, yorkensis), Diplophrys (species include archeri), Pyrrhosorus* (species include marinus), Sorodiplophrys* (species include stercorea), Chlamydomyxa* (species include labyrinthuloides, montand). (* = there is no current general consensus on the exact taxonomic placement of these genera).

It has been discovered herein that protists of the Ichthyosporea class are excellent sources for DPA(n-3). Accordingly, another preferred microorganism is a protist of the Ichthyosporea class. Examples of protists within the Ichthyosporea class include but are not limited to Ichthyophonida such as Creolimax fragrantissima, Sphaeroforma, Anurofeca richardsi, Psoropermium, Caullerya mesnili, Pirum gemmata, Abeoforma whisleri, Amoebidium, Paramoebidium, Eccrinales, and Icthyophonus, and Dermocystida such as Rhinosporidium seeberi, Dermocystidium percae, Sphaerothecum destruens, Amphibiothecum penneri, Amphibiocystidium ranae, and Dermocystidium salmonis. *Sphaeroforma arctica* is particularly preferred.

In some embodiments, the microorganism is selected from the group consisting of algae, bacteria, fungi and protists. In some embodiments, the microorganism is a protist within the Ichthyosporea class. In some embodiments, the microorganism is *Sphaeroforma arctica.* In other embodiments, the microorganism selected from the group consisting of Thraustochytriales, dinoflagellates, and Mucorales. In some embodiments, the microorganism is selected from the group consisting of Schizochytrium, Thraustochytrium, Crypthecodinium, and Mortierella.

In some embodiments, the microorganism is a genetically modified microorganism, wherein the genetic modification comprises the introduction of a polyketide synthase gene or a portion thereof.

Compositions comprising a microbial oil described herein are further provided. Examples of compositions comprising a microbial oil described herein include, but are not limited to, infant formulas, dietary supplements, nutritional supplements, food compositions, beverages, therapeutic drinks, nutritional drinks, cosmetic, pharmaceutical composition, etc.

Microbial strains capable of producing a microbial oil described herein are further provided, as are microbial oils produced by these microbial strains and compositions comprising the microbial oils. Microbial strains deposited with the Culture Collection of Algae and Protozoa under the accession number CCAP 3067/1, or a strain derived therefrom, are further provided, as are microbial oils produced by these microbial strains and compositions comprising the microbial oils.

### EXAMPLES

### EXAMPLE 1

### Strain selection

Several microbial strains were screened to identify strains that produce high levels of DPA(n-3). Microbial strains were fermented in 2X SDFMO medium, 1000 ppm Cl-, 10% CO₂, at 22.5°C. Total FAME profiles from selected strains at various time points are provided in Table 1.

**Table 1**

| **Strain** | **Harvest day** | **Dry weight (g/L)** | **% 16:0** | **% 18:0** | **% 18:1 n-9** | **% 20:2** | **% 20:4 ARA** | **% 20:5 n-3 EPA** | **% 22:5 n-3 DPA** | **% 22:6 n-3 DHA** | **% Fat** |
|---|---|---|---|---|---|---|---|---|---|---|---|
| MK2847 | 4 | 8.20 | 28.62 | 7.34 | 16.67 | 8.28 | 5.67 | 3.55 | 9.19 | 3.55 | 22.60 |
| | 6 | 16.11 | 20.48 | 6.88 | 27.42 | 5.93 | 7.94 | 3.46 | 6.51 | 1.98 | 37.00 |
| | 8 | 16.90 | 20.57 | 6.68 | 27.97 | 5.74 | 7.59 | 3.09 | 6.95 | 1.97 | 40.40 |
| MK2855 | 4 | 8.83 | 29.02 | 9.36 | 17.77 | 7.90 | 4.08 | 4.15 | 8.41 | 3.65 | 18.70 |
| | 6 | 12.14 | 22.67 | 8.61 | 25.20 | 6.84 | 5.28 | 3.71 | 7.24 | 2.85 | 27.60 |
| | 8 | 12.68 | 22.48 | 8.23 | 26.70 | 6.65 | 5.12 | 4.19 | 8.24 | 2.91 | 29.70 |
| MK2857 | 4 | 3.64 | 28.48 | 4.12 | 11.35 | 6.58 | 3.35 | 9.43 | 11.42 | 6.24 | 12.40 |
| | 6 | 11.42 | 22.93 | 8.32 | 26.32 | 6.63 | 5.80 | 3.94 | 7.40 | 2.88 | 27.10 |
| | 8 | 12.48 | 2 | 8.30 | 27.15 | 6.65 | 5.80 | 4.24 | 7.97 | 2.66 | 33.90 |
| MK2866 | 4 | 12.97 | 25.69 | 6.80 | 24.03 | 7.37 | 5.71 | 4.09 | 7.35 | 3.64 | 20.70 |
| | 6 | 16.04 | 21.68 | 6.11 | 28.93 | 7.49 | 5.39 | 4.56 | 7.91 | 3.05 | 30.00 |
| | 8 | 16.77 | 21.34 | 6.05 | 30.07 | 6.41 | 5.14 | 4.21 | 8.38 | 2.97 | 32.50 |
| MK2867 | 4 | 1.57 | 31.32 | 6.87 | 10.44 | 11.05 | 2.69 | 9.37 | 8.93 | 7.76 | 5.10 |
| | 6 | 9.38 | 23.02 | 6.96 | 25.11 | 9.29 | 4.41 | 6.11 | 9.04 | 4.72 | 18.70 |
| | 8 | 11.72 | 21.57 | 7.36 | 28.79 | 7.77 | 4.46 | 4.13 | 8.62 | 3.29 | 27.50 |

DPA(n-3) potency ranges from ~ 7-11% in top strains screened. Saturates are a majority of the fatty acids (FAs) produced. A comparison of DPA(n-3) production by all tested strains to top producing strains (MK 2847, MK 2855, MK 2857, MK 2866, and MK 2867) is shown in FIG. 1A & 1B, respectively.

Data from individual strains MK 2847, MK 2855, MK 2857, MK 2866, and MK 2867 are shown in FIGS. 2A-2D. FIG. 2A shows the median of % 22:5 DPA(n-3) over time. FIG. 2B shows the median of % 22:6 DHA (top panel) and the median of % 20:5 EPA(n-3) (bottom panel) over time. FIG. 2C shows the median of dry weight (g/L) (top panel) and the median of % fat as FAME (bottom panel) over time. FIG. 2D shows the median of fat titer (g/L) (top panel) and the median of DPA(n-3) titer (g/L) (bottom panel) over time.

### EXAMPLE 2

### Evaluation of fermentation conditions

DPA(n-3) and lipid production kinetics of the top four strains were evaluated under varying media conditions, CO2, and temperature as described in Table 2.

**Table 2**

| **Media³** | **CO₂ Level** | **Temp.** | **Harvest Time Points** | **Measurements Collected** | **N** | **# Flasks** | |
|---|---|---|---|---|---|---|---|
| **2X SDFMO, pH 7.5** | 10%¹ | 22.5°C | Days 4, 6, 8, 10 | Biomass, pH Glucose FAME | 3 | 48 | Standard screening condition |
| Standard medium with 1000 ppm Cl⁻ | | | | | | | |
| **2X SDFMO, pH 7.1** | Ambient 2 | 27°C | Days 5, 7, 9, 11 | Biomass, pH Glucose FAME | 3 | 48 | High temperature |
| Standard medium with 1000 ppm Cl⁻ | | | | | | | |
| **2X SDFMB, pH 7.5** | 10% | 22.5°C | Days 4, 6, 8, 10 | Biomass, pH Glucose FAME | 3 | 48 | Adjusted nutrients |
| Standard medium with 1000 ppm Cl⁻ | | | | | | | |
| **2X SDFMB, pH 7.1** | Ambient | 27°C | Days 5, 7, 9, 11 | Biomass, pH Glucose FAME | 3 | 48 | High temperature, adjusted nutrients |
| Standard medium with 1000 ppm Cl⁻ | | | | | | | |

| | | | | | | | |
|---|---|---|---|---|---|---|---|
| ¹ 10% CO2 - Improved FA profile; highlights DPA(n-3) differences ² Higher temperature requires ambient CO2 for growth | | | | | | | |

Growth and lipid production data comparing the different media at 10% CO₂ and at 22.5° C is illustrated in FIGS. 3A and 3B.

PUFA production data comparing the different media at 10% CO₂ and at 22.5° C is illustrated in FIGS. 4A and 4B. MK 2867 produced the highest % DPA(n-3) of all strains tested in this experiment (~10%) but all strains produced high levels (~8-9% DPA(n-3)). Low % DHA within acceptable range for all MK strains. Strain 122RT-100-6H3 produced higher % of DHA resulting in a DPA(n-3):DHA ratio greater than 1 but also accumulates a significantly higher % fat than the MK strains (~50% vs ~30%) and is a more efficient fat producer (~18% yield vs ~12-13% yield with MK strains).

FAME profile from the strains grown on 2X SDFMO, 1000 ppm Cl⁻, 10% CO₂, 22.5°C is provided in Table 3.

**Table 3**

| **Strain** | **Harvest day** | **Dry weight (g/L)** | **Fat titer (g/L)** | **% 20:5 n-3 EPA** | **% 22:5 n-3 DPA** | **% 22:6 n-3 DHA** | **% Fat** |
|---|---|---|---|---|---|---|---|
| MK 2857 | 4 | 14.60 | 4.27 | 3.76 | 7.89 | 2.95 | 29.23 |
| | 6 | 15.18 | 4.86 | 3.60 | 8.36 | 3.04 | 32.10 |
| | 8 | 16.05 | 5.32 | 3.40 | 8.36 | 2.74 | 33.17 |
| | 10 | 7.94 | 2.41 | 2.80 | 5.80 | 2.66 | 30.20 |
| MK 2866 | 4 | 10.70 | 3.01 | 4.36 | 7.91 | 3.13 | 27.95 |
| | 6 | 14.05 | 4.54 | 4.20 | 8.74 | 3.10 | 32.57 |
| | 8 | 15.15 | 5.49 | 4.27 | 8.04 | 2.70 | 36.23 |
| | 10 | 17.89 | 6.66 | 4.31 | 8.13 | 2.85 | 37.20 |
| MK 2867 | 4 | 13.35 | 4.09 | 4.30 | 8.42 | 2.97 | 30.67 |
| | 6 | 13.44 | 3.97 | 4.41 | 9.85 | 3.84 | 29.20 |
| | 8 | 15.11 | 5.13 | 4.17 | 9.71 | 3.11 | 33.73 |
| | 10 | 13.05 | 3.96 | 4.41 | 9.81 | 3.84 | 30.03 |
| 122RT-100-6H3 | 4 | 9.70 | 4.29 | 18.32 | 8.70 | 43.34 | 44.27 |
| | 6 | 9.99 | 5.18 | 17.20 | 6.72 | 46.52 | 51.93 |
| | 8 | 9.70 | 3.96 | 14.33 | 7.39 | 50.06 | 40.63 |
| | 10 | 8.66 | 2.95 | 12.99 | 6.76 | 54.37 | 34.07 |

Fermentation at 27° C and at ambient CO₂ resulted in poor growth in both media compared to 22.5° C, with only slight increase in DPA(n-3) seen in some strains.

Comparison of data collected in Example 1 with this data demonstrates that DPA(n-3) production tends to be highly variable, especially in the MK 2857 strain. However, DPA(n-3) % is consistent between both experiments for the MK 2867 strain.

### EXAMPLE 3

### Follow-up screening

This screening evaluates and compares the growth, fatty acid profile, and lipid production kinetics of 122RT-100-6H3, MK 2867, and various production strains for potential use of DPA(n-3) oil in compositions for human or animal consumption, including infant formula compositions.

Fermentation conditions were similar to Example 2 (2X SDFMO media, pH 7.5, 10% CO₂, 22.5° C).

Growth and lipid production kinetics are illustrated in FIGS. 5A and 5B. GO 6.117 and 9.1.5.5 strains have higher % fat and carbon conversion efficiency than strains MK 2857, MK 2867 + clones.

PUFA profiles for the tested strains are illustrated in FIGS. 6A-6D. Strain MK 2867 (and its clones MK 2867-1 and MK 2867-6), and MK 2857 produce higher % DPA(n-3) than GO 6.117 and 9.1.5.5 strains. MK 2867 with 10% DPA(n-3) displayed the highest potency of all strains. Strain 122RT-100-6H3 is unique due to moderate % fat with high DPA, EPA, moderate DPA(n-3)%.

Because Strain 9.1.5.5 is typically grown on SDFMB media, performance of the strain on both SDFMO and SDFMB was compared. Strain 9.1.5.5 performance is slightly higher in SDFMB vs SDFMO, but trends are comparable in all key metrics (biomass, fat titer, % fat, FA profile) thus validating the data presented above comparing strain 9.1.5.5 and other strains grown on SDFMO media.

Comparison of the MK 2867 clones (MK 2867-1 and MK 2867-6) to the parent strain is illustrated in FIG. 7A and 7B.

The citation of any reference is for its disclosure prior to the filing date and should not be construed as an admission that the present disclosure is not entitled to antedate such reference by virtue of prior invention.

The foregoing embodiments are presented by way of example only; the scope of the present disclosure is to be limited only by the following claims.

## Claims

1. An infant formula composition, wherein the composition comprises long chain polyunsaturated fatty acids (LC-PUFAs) and wherein, when ready for consumption by the infant, the LC-PUFAs comprise docosapentaenoic acid n-3 (DPA(n-3)) and docosahexaenoic acid (DHA) in a ratio of DPA(n-3):DHA greater than 0.2, and wherein the LC-PUFAs are supplied in a source oil, wherein the source oil comprises DPA(n-3) in an amount of at least about 7% by weight.

2. The composition of claim 1, wherein the ratio of DPA(n-3):DHA is between 0.2:1 and 1:1.

3. The composition of claim 1, wherein the ratio of DPA(n-3):DHA is between 0.3:1 and 0.8:1.

4. The composition of any preceding claim, wherein the source oil comprises DPA(n-3) in an amount between about 7% to about 12% by weight.

5. The composition of any preceding claim, wherein the source oil comprises DPA(n-3) in an amount between about 8% to about 11% by weight.

6. The composition of any preceding claim, wherein the ratio of DPA(n-3):DHA greater than 0.2 is obtained by mixing a first plant, fish, or microbial oil, or combinations thereof, with a second microbial oil having a DHA:DPA(n-3):EPA ratio of 0.01-0.7:1:0.01-0.7, optionally having a DHA:DPA(n-3):EPA ratio of 0.2-0.7:1:0.2-0.7.

7. The composition of claim 6, wherein the second microbial oil is obtained from a microorganism selected from the group consisting of algae, bacteria, fungi and protists.

8. The composition of claim 7, wherein the second microbial oil is obtained from a microorganism of the Ichthyosporea class.

9. The composition of claim 8, wherein the microorganism is *Sphaeroforma arctica.*

10. The composition of any one of claims 7-9, wherein the first oil is obtained from a microorganism selected from the group consisting of Thraustochytriales, dinoflagellates, and Mortierella.

11. The composition of claim 10, wherein the microorganism is selected from the group consisting of Schizochytrium, Thraustochytrium, and Crypthecodinium.

12. The composition of any preceding claim, prepared as product form selected from the group consisting of reconstitutable powders, ready-to-feed liquids, and dilutable liquid concentrates.

13. The composition of any preceding claim, prepared as reconstitutable powder.

## Patentansprüche

1. Säuglingsanfangsnahrungszusammensetzung, wobei die Zusammensetzung langkettige mehrfach ungesättigte Fettsäuren (LC-PUFA) umfasst und wobei, wenn sie für den Säugling verbrauchsfertig ist, die LC-PUFA Docosapentaensäure-n-3 (DPA(n-3)) und Docosahexaensäure (DHA) in einem Verhältnis von DPA(n-3):DHA von mehr als 0,2 umfassen, und wobei die LC-PUFA in einem Quellöl zugeführt werden, wobei das Quellöl DPA(n-3) in einer Menge von mindestens etwa 7 Gew.-% umfasst.

2. Zusammensetzung nach Anspruch 1, wobei das Verhältnis von DPA(n-3):DHA zwischen 0,2:1 und 1:1 liegt.

3. Zusammensetzung nach Anspruch 1, wobei das Verhältnis von DPA(n-3):DHA zwischen 0,3:1 und 0,8:1 liegt.

4. Zusammensetzung nach einem vorhergehenden Anspruch, wobei das Quellöl DPA(n-3) in einer Menge zwischen etwa 7 Gew.-% bis etwa 12 Gew.-% umfasst.

5. Zusammensetzung nach einem vorhergehenden Anspruch, wobei das Quellöl DPA(n-3) in einer Menge zwischen etwa 8 Gew.-% bis etwa 11 Gew.-% umfasst.

6. Zusammensetzung nach einem vorhergehenden Anspruch, wobei das Verhältnis von DPA(n-3):DHA von mehr als 0,2 durch Mischen eines ersten Pflanzen-, Fisch- oder mikrobiellen Öls oder Kombinationen davon mit einem zweiten mikrobiellen Öl mit einem DHA:DPA(n-3):EPA-Verhältnis von 0,01-0,7:1:0,01-0,7, optional mit einem DHA:DPA(n-3):EPA-Verhältnis von 0,2-0,7:1:0,2-0,7 erhalten wird.

7. Zusammensetzung nach Anspruch 6, wobei das zweite mikrobielle Öl von einem Mikroorganismus erhalten wird, der aus der Gruppe bestehend aus Algen, Bakterien, Pilzen und Protisten ausgewählt ist.

8. Zusammensetzung nach Anspruch 7, wobei das zweite mikrobielle Öl von einem Mikroorganismus der Ichthyosporea-Klasse erhalten wird.

9. Zusammensetzung nach Anspruch 8, wobei der Mikroorganismus *Sphaeroforma arctica* ist.

10. Zusammensetzung nach einem der Ansprüche 7-9, wobei das erste Öl von einem Mikroorganismus erhalten wird, der aus der Gruppe bestehend aus Thraustochytriales, Dinoflagellaten und Mortierella ausgewählt ist.

11. Zusammensetzung nach Anspruch 10, wobei der Mikroorganismus aus der Gruppe bestehend aus Schizochytrium, Thraustochytrium und Crypthecodinium ausgewählt ist.

12. Zusammensetzung nach einem vorhergehenden Anspruch, als Produktform hergestellt, die aus der Gruppe bestehend aus rekonstituierbaren Pulvern, fütterfertigen Flüssigkeiten und verdünnbaren flüssigen Konzentraten ausgewählt ist.

13. Zusammensetzung nach einem vorhergehenden Anspruch, als rekonstituierbares Pulver hergestellt.

## Revendications

1. Composition de préparation pour nourrissons, dans laquelle la composition comprend des acides gras polyinsaturés à longue chaîne (AGPI-LC) et dans laquelle, lorsqu'elle est prête à être consommée par le nourrisson, les AGPI-LC comprennent de l'acide docosapentaénoïque n-3 (DPA(n-3)) et de l'acide docosahexaénoïque (DHA) dans un rapport DPA(n-3):DHA supérieur à 0,2, et dans laquelle les AGPI-LC sont fournis sous la forme d'une huile source, dans laquelle l'huile source comprend du DPA(n-3) en une quantité d'au moins environ 7 % en poids.

2. Composition de la revendication 1, dans laquelle le rapport DPA(n-3):DHA est compris entre 0,2:1 et 1:1.

3. Composition de la revendication 1, dans laquelle le rapport DPA(n-3):DHA est compris entre 0,3:1 et 0,8:1.

4. Composition de l'une quelconque des revendications précédentes, dans laquelle l'huile source comprend du DPA(n-3) en une quantité comprise entre environ 7 % et environ 12 % en poids.

5. Composition de l'une quelconque des revendications précédentes, dans laquelle l'huile source comprend du DPA(n-3) en une quantité comprise entre environ 8 % et environ 11 % en poids.

6. Composition de l'une quelconque des revendications précédentes, dans laquelle le rapport DPA(n-3):DHA supérieur à 0,2 est obtenu en mélangeant une première huile végétale, de poisson ou microbienne, ou leurs combinaisons, avec une seconde huile microbienne présentant un rapport DHA:DPA(n-3):EPA de 0,01-0,7:1:0,01-0,7, présentant éventuellement un rapport DHA:DPA(n-3):EPA de 0,2-0,7:1:0,2-0,7.

7. Composition de la revendication 6, dans laquelle la seconde huile microbienne est obtenue à partir d'un micro-organisme choisi dans le groupe constitué par les algues, les bactéries, les champignons et les protistes.

8. Composition de la revendication 7, dans laquelle la seconde huile microbienne est obtenue à partir d'un micro-organisme de la classe des Ichthyosporea.

9. Composition de la revendication 8, dans laquelle le micro-organisme est *Sphaeroforma arctica.*

10. Composition de l'une quelconque des revendications 7 à 9, dans laquelle la première huile est obtenue à partir d'un micro-organisme choisi dans le groupe constitué par les Thraustochytriales, les dinoflagellés et Mortierella.

11. Composition de la revendication 10, dans laquelle le micro-organisme est choisi dans le groupe constitué par Schizochytrium, Thraustochytrium et Crypthecodinium.

12. Composition de l'une quelconque des revendications précédentes, préparée sous une forme de produit choisie dans le groupe constitué par les poudres reconstituables, les liquides prêts à l'emploi et les concentrés liquides diluables.

13. Composition de l'une quelconque des revendications précédentes, préparée sous forme de poudre reconstituable.
